# EUROPEAN PATENT APPLICATION

(11) **EP 3 922 976 A1**
(43) Date of publication of application: **15.12.2021**
(21) Application number: 20752723.5
(22) Date of filing: 06.02.2020
(51) Int. Cl.: G01N 5/02, G01N 27/12

(54) **SENSOR**

(30) Priority: 08.02.2019 JP 2019021223; 29.08.2019 JP 2019156913; 09.12.2019 JP 2019221949
(71) Applicant: Asahi Kasei Kabushiki Kaisha, Tokyo 1000006 (JP)
(72) Inventor: TSUJIMOTO, Hiroyuki, Tokyo 100-0006 (JP); YOSHIDA, Makoto, Tokyo 100-0006 (JP); KIMURA, Ippei, Tokyo 100-0006 (JP); YAMAKAWA, Natsumi, Tokyo 100-0006 (JP)
(74) Representative: Hoffmann Eitle
(86) International application number: PCT/JP2020/004554
(87) International publication number: WO 2020/162538

(57) **Abstract**

A sensor having an acceptor to which a substance to be detected adheres,
wherein log domain area ratio A is -4.1 or more, the log domain area ratio A providing a total occupancy maximum in a distribution of total occupancies against log domain area ratios calculated by subjecting the acceptor to the following measurement:

[Measurement]

In an image obtained by observing under a scanning electron microscope a surface or a cross section at an arbitrary position of the acceptor, quadtree spatial partitioning is performed in order to discriminate between void portions and dense portions of the surface or the cross section; a quadrangle group corresponding to the dense portions obtained by the quadtree spatial partitioning is classified on an area basis; and the log domain area ratio and the total occupancy are calculated as follows:

(Log domain area ratio)

A value determined by dividing each area of the quadrangle group by the area of the image is regarded as a domain area ratio, and a common logarithm thereof is taken as the log domain area ratio, and

(Total occupancy)

A value, which is represented by a ratio of a total area value of quadrangles having the same area in the quadrangle group to the area of the image being defined as 1, is regarded as the total occupancy.

## Description

### Technical Field

The present invention relates to a sensor.

### Background Art

In general, sensors have a detection part (acceptor) capable of high-sensitively and high-selectively detecting a target substance to be detected. A wide variety of substances such as self-assembled films, monomolecular films, DNA/RNA, proteins, antigens/antibodies, and polymers are used as such acceptors. In the field of olfactory or gas sensors, novel applications for smell management or the like in breath tests, hotels, automobiles, or factories have been expected with recent development of AI or IoT. If ethanol or aldehyde derived from the anaerobic respiration of food is traceable, the quality of food products can be managed with sensors.

Patent Literature 1 describes a sensor which detects an analyte molecule from change in physical parameter caused by the adsorption of the analyte molecule by a porous material or a granular material, wherein a sensor body of physical parameter detection type is covered with the porous material or the granular material.

Patent Literature 2 describes a fuel oil identification sensor comprising a sensor body which detects change in physical parameter, and a hydrocarbon group-modified fine particle having a particle size of 1 mm or smaller, wherein the sensor is provided with an acceptor layer which covers the sensor body surface.

Patent Literature 3 describes a quartz crystal microbalance (QCM) gas sensor which detects VOC gas through the use of the adsorptive characteristics between the target gas to be measured and a layered inorganic compound particle, wherein the gas sensor has a structure where a porous organic-inorganic hybrid particle in which an organic residue is inserted and fixed between layers of a layered inorganic compound is laminated as a detection membrane on a quartz crystal electrode.

Patent Literature 4 describes a gas detection element which has a gas-sensitive part and detects a gas to be detected on the basis of change in electrical resistance of the gas-sensitive part, wherein the gas-sensitive part is cerium oxide, and the gas to be detected is at least any gas of an oxygen-containing organic compound and a sulfur-containing compound.

Patent Literature 5 describes a moisture-sensitive sensor having a sensitive part made of a sensitive material containing a flexible high-molecular material insoluble in water mixed with a soluble polymer having responsiveness to humidity, wherein the sensor detects humidity or dew condensation by measuring change in conductivity of the sensitive part.

Patent Literature 6 describes a humidity sensor prepared by forming a moisture-sensitive membrane on a piezoelectric element via a thin-membrane electrode, wherein the moisture-sensitive membrane is a thin membrane of porous glass.

### Citation List

### Patent Literature

Patent Literature 1: International Publication No. WO 2016/121155
Patent Literature 2: International Publication No. WO 2017/098862
Patent Literature 3: Japanese Patent Laid-Open No. 2011-080983
Patent Literature 4: Japanese Patent Laid-Open No. 2010-002335
Patent Literature 5: Japanese Patent Laid-Open No. 11-101766
Patent Literature 6: Japanese Patent Laid-Open No. 62-291541

### Summary of Invention

### Technical Problem

The technique described in Patent Literature 1 achieves high sensitivity, selectivity, and stability, whereas there are large limitations on environments where the technique can be used as an olfactory sensor because electrical signals attenuate drastically in an atmosphere other than a dry atmosphere. In the technique described in Patent Literature 2, a substance to be detected is limited to a highly hydrophobic gas.

The technique described in Patent Literature 3 achieves a VOC gas sensor having favorable response recovery properties and reproducibility, whereas the technique cannot be reproducibly used under conditions other than dry-restricted conditions because the adsorption of humidity in air creates an interfering gas in an environment other than under drying so that the adsorption of a target substance to be detected is inhibited.

In the technique described in Patent Literature 4, a substance to be detected is limited to only the gases described above.

In the techniques described in Patent Literatures 5 and 6, a substance to be detected is limited to only humidity (water).

The present invention has been made in light of the problems of the conventional techniques described above. An object of the present invention is to provide a sensor that is capable of high-sensitively measuring a highly hydrophilic gas such as ethanol or aldehyde even under conditions other than dry-restricted conditions and is further capable of continuously measuring targets without deteriorating an acceptor even after reflow treatment and after a long period of use.

### Solution to Problem

The present inventors have conducted diligent studies and experiments to attain the object, and consequently completed the present invention by finding that, unexpectedly, the object can be attained.

Specifically, the present invention encompasses the following aspects.
[1] A sensor comprising an acceptor to which a substance to be detected adheres,
   wherein log domain area ratio A is -4.1 or more, the log domain area ratio providing a total occupancy maximum in a distribution of total occupancies against log domain area ratios calculated by subjecting the acceptor to the following measurement:

### [Measurement]

In an image obtained by observing under a scanning electron microscope a surface or a cross section at an arbitrary position of the acceptor, quadtree spatial partitioning is performed in order to discriminate between void portions and dense portions of the surface or the cross section; a quadrangle group corresponding to the dense portions obtained by the quadtree spatial partitioning is classified on an area basis; and the log domain area ratio and the total occupancy are calculated as follows:

### (Log domain area ratio)

A value determined by dividing each area of the quadrangle group by the area of the image is regarded as a domain area ratio, and a common logarithm thereof is taken as the log domain area ratio, and

### (Total occupancy)

A value, which is represented by a ratio of a total area value of quadrangles having the same area in the quadrangle group to the area of the image being defined as 1, is regarded as the total occupancy.
[2] The sensor according to [1], wherein the log domain area ratio A is -3.5 or more.
[3] A sensor comprising an acceptor to which a substance to be detected adheres,
   wherein log domain area ratio B is -4.1 or more, the log domain area ratio B providing a cumulative total occupancy of 60% in an ascending order of log domain area ratios in a distribution of total occupancies against log domain area ratios calculated by subjecting the acceptor to the following measurement:

### [Measurement]

In an image obtained by observing under a scanning electron microscope a surface or a cross section at an arbitrary position of the acceptor, quadtree spatial partitioning is performed in order to discriminate between void portions and dense portions of the surface or the cross section; a quadrangle group corresponding to the dense portions obtained by the quadtree spatial partitioning is classified on an area basis; and the log domain area ratio and the total occupancy are calculated as follows:

### (Log domain area ratio)

A value determined by dividing each area of the quadrangle group by the area of the image is regarded as a domain area ratio, and a common logarithm thereof is taken as the log domain area ratio, and

### (Total occupancy)

A value, which is represented by a ratio of a total area value of quadrangles having the same area in the quadrangle group to the area of the image being defined as 1, is regarded as the total occupancy.
[4] The sensor according to [3], wherein the log domain area ratio B is -3.5 or more.
[5] The sensor according to any of [1] to [4], wherein the acceptor comprises an organic-inorganic hybrid.
[6] The sensor according to [5], wherein the organic-inorganic hybrid is represented by RSiO_{3/2} wherein the R represents an organic functional group.
[7] A sensor comprising an acceptor to which a substance to be detected adheres, wherein
   the acceptor comprises an organic-inorganic hybrid having a structure represented by RSiO_{3/2} wherein the R represents an organic functional group, and
   a void ratio as a volume fraction of the acceptor is 10% or less.
[8] The sensor according to [7], wherein the void ratio is 5% or less.
[9] The sensor according to [7] or [8], wherein the void ratio is 3% or less.
[10] A sensor comprising an acceptor to which a substance to be detected adheres,
   wherein the acceptor comprises an organic-inorganic hybrid having a structure represented by RSiO_{3/2} wherein the R represents an organic functional group,
   the acceptor comprises a first component having a weight-average molecular weight of 800 to 30000, and a second component having a weight-average molecular weight of 30000 to 2000000, and
   in a chromatogram obtained by measuring the acceptor by gel permeation chromatography, a peak area of the second component is 0.01% to 55% with respect to a total peak area of the first component and the second component.
[11] The sensor according to [10], wherein the acceptor further comprises a filler, and the second component is derived from the filler.
[12] The sensor according to any of [1] to [9], wherein the acceptor further comprises a filler.
[13] The sensor according to [11] or [12], wherein the filler is an inorganic filler.
[14] The sensor according to any of [11] to [13], wherein the filler is a metal oxide.
[15] The sensor according to any of [11] to [14], wherein the filler is silica.
[16] The sensor according to any of [11] to [15], wherein a shape of the filler is a spherical shape or a chain-like shape.
[17] The sensor according to any of [6] to [16], wherein the organic functional group has an aromatic ring.
[18] The sensor according to [17], wherein the aromatic ring is bonded directly to the Si atom in the organic-inorganic hybrid.
[19] The sensor according to [17] or [18], wherein the organic functional group has a copolymer structure of the aromatic ring and a non-aromatic ring.
[20] The sensor according to any of [6] to [19], wherein the organic functional group has at least one atom selected from the group consisting of a halogen atom, a nitrogen atom, an oxygen atom, a phosphorus atom, a sulfur atom and a boron atom.
[21] The sensor according to any of [1] to [20], wherein an amount of the organic functional group in the organic-inorganic hybrid is 75% by mass or less.
[22] The sensor according to any of [1] to [21], wherein a largest thickness of the acceptor is 30 µm or smaller.
[23] The sensor according to any of [1] to [22], wherein the sensor is a membrane-type surface stress sensor.

### Advantageous Effects of Invention

The present invention can provide a sensor that is capable of high-sensitively measuring a highly hydrophilic gas such as ethanol or aldehyde even in a humid state and is further capable of continuously measuring targets without deteriorating an acceptor even after reflow treatment and after a long period of use.

### Brief Description of Drawings

[Figure 1] Figure 1 shows an example of an optical microscope photograph of a chip coated with an acceptor.
[Figure 2] Figure 2 shows a scanning electron microscope (SEM) image of an acceptor surface in Example 1.
[Figure 3] Figure 3 shows a SEM image of a cross section in Example 1.
[Figure 4] Figure 4 shows an image obtained by observing the acceptor of Example 1 under a confocal microscope, and a film thickness distribution.
[Figure 5] Figure 5 shows responsiveness to ethanol at a humidity of 0% or 30% in Example 1.
[Figure 6] Figure 6 shows a SEM image of an acceptor surface in Comparative Example 1.
[Figure 7] Figure 7 shows responsiveness to ethanol at a humidity of 0% or 30% in Comparative Example 1.
[Figure 8] Figure 8 shows results of microscopic ATR spectra of Example 1 and a MSS sensor for a control.
[Figure 9] Figure 9 shows a scanning electron microscope (SEM) image of an acceptor surface in Example 1.
[Figure 10] Figure 10 shows cross-sectional images at arbitrary 3 points (top to bottom) of the acceptor of Example 1 (left), images obtained by binarizing the cross-sectional images (central), and images obtained by quadtree spatial partitioning (right).
[Figure 11] Figure 11 shows a graph showing a plot of total occupancies against log domain area ratios of the acceptor of Example 1.
[Figure 12] Figure 12 shows a graph showing a plot of cumulative total occupancies against log domain area ratios of the acceptor of Example 1.
[Figure 13] Figure 13 shows a SEM image of an acceptor surface in Example 2.
[Figure 14] Figure 14 shows cross-sectional images at arbitrary 5 points (top to bottom) of the acceptor of Example 2 (left), images obtained by binarizing the cross-sectional images (central), and images obtained by quadtree spatial partitioning (right).
[Figure 15] Figure 15 shows a graph showing a plot of total occupancies against log domain area ratios of the acceptor of Example 2.
[Figure 16] Figure 16 shows a graph showing a plot of cumulative total occupancies against log domain area ratios of the acceptor of Example 2.
[Figure 17] Figure 17 shows a SEM image of an acceptor surface in Comparative Example 1.
[Figure 18] Figure 18 shows cross-sectional images at arbitrary 3 points (top to bottom) of the acceptor surface of Comparative Example 1 (left), images obtained by binarizing the cross-sectional images (central), and images obtained by quadtree spatial partitioning (right).
[Figure 19] Figure 19 shows a graph showing a plot of total occupancies against log domain area ratios of the acceptor of Comparative Example 1.
[Figure 20] Figure 20 shows a graph showing a plot of cumulative total occupancies against log domain area ratios of the acceptor of Comparative Example 1.
[Figure 21] Figure 21 shows results of gel permeation chromatography (GPC) in Example 1.
[Figure 22] Figure 22 shows results of GPC in Example 2.

### Description of Embodiments

Hereinafter, an embodiment for carrying out the present invention (hereinafter, referred to as the "present embodiment") will be described in detail. However, the present invention is not limited by the present embodiment. Various changes or modifications can be made therein without departing from the spirit of the present invention.

The sensor according to the first aspect of the present embodiment (hereinafter, also referred to as the "first sensor") is a sensor comprising an acceptor to which a substance to be detected adheres, wherein log domain area ratio A is -4.1 or more, the log domain area ratio A providing a total occupancy maximum in a distribution of total occupancies against log domain area ratios calculated by subjecting the acceptor to the following measurement:

### [Measurement]

In an image obtained by observing under a scanning electron microscope a surface or a cross section at an arbitrary position of the acceptor, quadtree spatial partitioning is performed in order to discriminate between void portions and dense portions of the surface or the cross section; a quadrangle group corresponding to the dense portions obtained by the quadtree spatial partitioning is classified on an area basis; and the log domain area ratio and the total occupancy are calculated as follows:

### (Log domain area ratio)

A value determined by dividing each area of the quadrangle group by the area of the image is regarded as a domain area ratio, and a common logarithm thereof is taken as the log domain area ratio, and

### (Total occupancy)

A value, which is represented by a ratio of a total area value of quadrangles having the same area in the quadrangle group to the area of the image being defined as 1, is regarded as the total occupancy.

The first sensor thus configured can high-sensitively measure a highly hydrophilic gas such as ethanol or aldehyde even in a humid state and can further continuously measure targets without deteriorating the acceptor even after reflow treatment and after a long period of use.

The sensor according to the second aspect of the present embodiment (hereinafter, also referred to as the "second sensor") is a sensor comprising an acceptor to which a substance to be detected adheres, wherein log domain area ratio B is -4.1 or more, the log domain area ratio B providing a cumulative total occupancy of 60% in an ascending order of log domain area ratios in a distribution of total occupancies against log domain area ratios calculated by subjecting the acceptor to the following measurement:

### [Measurement]

In an image obtained by observing under a scanning electron microscope a surface or a cross section at an arbitrary position of the acceptor, quadtree spatial partitioning is performed in order to discriminate between void portions and dense portions of the surface or the cross section; a quadrangle group corresponding to the dense portions obtained by the quadtree spatial partitioning is classified on an area basis; and the log domain area ratio and the total occupancy are calculated as follows:

### (Log domain area ratio)

A value determined by dividing each area of the quadrangle group by the area of the image is regarded as a domain area ratio, and a common logarithm thereof is taken as the log domain area ratio, and

### (Total occupancy)

A value, which is represented by a ratio of a total area value of quadrangles having the same area in the quadrangle group to the area of the image being defined as 1, and the resulting value is regarded as the total occupancy.

The second sensor thus configured can high-sensitively measure a highly hydrophilic gas such as ethanol or aldehyde even in a humid state and can further continuously measure targets without deteriorating the acceptor even after reflow treatment and after a long period of use.

The sensor according to the third aspect of the present embodiment (hereinafter, also referred to as the "third sensor") is a sensor comprising an acceptor to which a substance to be detected adheres, wherein the acceptor comprises an organic-inorganic hybrid having a structure represented by RSiO_{3/2} wherein the R represents an organic functional group, and a void ratio as a volume fraction of the acceptor is 10% or less.

The third sensor thus configured can high-sensitively measure a highly hydrophilic gas such as ethanol or aldehyde even in a humid state and can further continuously measure targets without deteriorating the acceptor even after reflow treatment and after a long period of use.

The sensor according to the fourth aspect of the present embodiment (hereinafter, also referred to as the "fourth sensor") is a sensor comprising an acceptor to which a substance to be detected adheres, wherein the acceptor comprises an organic-inorganic hybrid having a structure represented by RSiO_{3/2} wherein the R represents an organic functional group, the acceptor comprises a first component having a weight-average molecular weight of 800 to 30000, and a second component having a weight-average molecular weight of 30000 to 2000000, and in a chromatogram obtained by measuring the acceptor by gel permeation chromatography, a peak area of the second component is 0.01% to 55% with respect to a total peak area of the first component and the second component.

The fourth sensor thus configured can high-sensitively measure a highly hydrophilic gas such as ethanol or aldehyde even in a humid state and can further continuously measure targets without deteriorating the acceptor even after reflow treatment and after a long period of use.

Hereinafter, the term "sensor of the present embodiment" encompasses the first sensor, the second sensor, the third sensor and the fourth sensor, and the term "acceptor according to the present embodiment" encompasses the acceptor for the first sensor, the acceptor for the second sensor, the acceptor for the third sensor and the acceptor for the fourth sensor, unless otherwise specified.

The sensor of the present embodiment is not particularly limited and comprises, for example, a sensor body of physical parameter detection type. Examples of the physical parameter include, but are not particularly limited to, surface stress, stress, force, surface tension, pressure, mass, elasticity, Young's modulus, Poisson's ratio, resonance frequency, frequency, volume, thickness, viscosity, density, magnetic force, magnetic quantity, magnetic field, magnetic flux, magnetic flux density, electrical resistance, quantity of electricity, permittivity, electric power, electric field, electric charge, current, voltage, potential, mobility, electrostatic energy, capacitance, inductance, reactance, susceptance, admittance, impedance, conductance, plasmon, refractive index, luminance and temperature, and other various physical parameters. The sensor of the present embodiment is, for example, a sensor having an acceptor (also called "acceptor layer" or "sensitive membrane" according to a shape, etc. in the present technical field) disposed directly on the sensor body. Specifically, the sensor of the present embodiment can detect, through the sensor body, change in physical parameter caused in the acceptor by the adsorption and internal diffusion of a substance to be detected (analyte molecule) by the acceptor. As described above, the sensor body that may be used in the sensor of the present embodiment is not particularly limited as long as the sensor detects change caused in the acceptor by the adsorption and internal diffusion of a substance to be detected by the acceptor disposed on the surface of the sensor body.

In the case of applying the sensor of the present embodiment to, for example, a surface stress sensor (e.g., an olfactory sensor), a substance to be detected is adsorbed onto the acceptor (sensitive membrane) covering at least a portion of the surface of the sensor body, and further internally diffused into the acceptor so that the body detects change in stress caused in the acceptor and outputs signals.

For example, a QCM apparatus may be utilized as another type of sensor body. The QCM apparatus is a mass sensor that measures minute change in mass through the use of the property of decreasing resonance frequency according to the mass, viscoelasticity, or the like of an adsorbate upon adsorption of a substance onto a quartz crystal electrode surface to which an alternating electric field has been applied. This apparatus is capable of *in-situ* measurement. In the case of applying the sensor of the present embodiment to the QCM apparatus, the acceptor according to the present embodiment is formed, for example, on the surface of an electrode. A substance to be detected is adsorbed onto this acceptor so that the sensor body detects change in mass caused thereby and outputs signals. Various conductive materials are applicable to the electrode of QCM. In the case of imparting conductivity to the acceptor according to the present embodiment, this acceptor can also be used as the electrode of QCM.

The term "adsorption" used in the present specification is meant to include a phenomenon in which at the interface of a certain object, another substance (serving as an adsorbate) has a higher concentration than that of its surroundings, and includes not only physical adsorption but chemical adsorption through a chemical bond or a biochemical effect.

### [Acceptor]

The acceptor according to the present embodiment can induce change in signal by the adsorption and internal diffusion of an adsorbate (substance to be detected), for example, an odor molecule or a gas molecule. Particularly, an acceptor in a membrane shape is also referred to as a "sensitive membrane".

The constituents of the acceptor in the first sensor and the acceptor in the second sensor are not particularly limited as long as a substance to be detected adheres thereto and log domain area ratio A and log domain area ratio B, respectively, fall within predetermined ranges. Various materials are applicable according to the substance to be detected. The acceptor in the first sensor and the acceptor in the second sensor preferably comprise an organic-inorganic hybrid and/or a filler mentioned later, from the viewpoint of high-sensitively sensing a highly hydrophilic gas such as ethanol or aldehyde even under high humidity, and further from the viewpoint of maintaining the performance of the acceptor even after reflow treatment and after a long period of use.

The acceptor in the first sensor, when subjected to the measurement mentioned above, has log domain area ratio A of -4.1 or more. When the log domain area ratio A is -4.1 or more, the first sensor can high-sensitively measure a highly hydrophilic gas such as ethanol or aldehyde even in a humid state and can further continuously measure targets without deteriorating the acceptor even after reflow treatment and after a long period of use. In the first sensor, the log domain area ratio A is preferably -3.5 or more from the viewpoint of membrane stability, more preferably -3.3 or more from the viewpoint of sensitivity, further preferably -3.2 or more, from the viewpoint of humidity resistance. The first sensor can satisfy the above numeric range of the log domain area ratio A as to at least one surface or cross section at an arbitrary position of the acceptor. From the viewpoint of enhancing the effects mentioned above,
it is preferred that the largest value of the log domain area ratio A among surfaces or cross sections at arbitrary 3 or more points (3 or more fields of view) of the acceptor should satisfy the above numeric range, and it is more preferred that the largest value of the log domain area ratio A among 5 or more fields of view should satisfy the above numeric range. The log domain area ratio A can be adjusted to the above range, for example, by adjusting the ratio between a filler mentioned later and a structure represented by RSiO_{3/2} or the amount of an organic functional group.

For example, in the case of observing an acceptor cross section, the log domain area ratio and the total occupancy are values determined as follows: the acceptor is cut in the thickness direction or the width direction, and the obtained cross section is observed under SEM, for example, at a 100000× magnification. In the obtained SEM image, quadtree spatial partitioning is performed in order to discriminate between void portions and dense portions of the cross section. For pretreatment of the quadtree spatial partitioning, a threshold is defined on a small domain basis of the SEM image, and bright parts and dark parts are binarized by adaptive threshold processing. Subsequently, the binarized image is subjected to 3 × 3 spatial filtering to clarify the dark parts (void portions). Then, the quadtree spatial partitioning is carried out, and partitioning is continued until the number of black pixels (void pixels) contained in each domain of the partitions becomes 1. The number and area of dense portions free from the void pixels are determined. In this way, a quadrangle group corresponding to the dense portions is classified on an area basis, and the log domain area ratio and the total occupancy are calculated. The methods for calculating the log domain area ratio and the total occupancy are as mentioned above. More specifically, the log domain area ratio and the total occupancy can be determined by methods described in Examples mentioned later.

The acceptor in the second sensor, when subjected to the measurement mentioned above, has log domain area ratio B of -4.1 or more. When the log domain area ratio B is -4.1 or more, the second sensor can high-sensitively measure a highly hydrophilic gas such as ethanol or aldehyde even in a humid state and can further continuously measure targets without deteriorating the acceptor even after reflow treatment and after a long period of use. Furthermore, adhesiveness to the sensor body is also favorable. In the second sensor, the log domain area ratio B is preferably -3.5 or more from the viewpoint of sensitivity, more preferably -3.3 or more from the viewpoint of humidity resistance. The second sensor can satisfy the above numeric range of the log domain area ratio B as to at least one surface or cross section at an arbitrary position of the acceptor. From the viewpoint of enhancing the effects mentioned above, it is preferred that the largest value of the log domain area ratio B among surfaces or cross sections at arbitrary 3 or more points (3 or more fields of view) of the acceptor should satisfy the above numeric range, and it is more preferred that the largest value of the log domain area ratio B among 5 or more fields of view should satisfy the above numeric range. The log domain area ratio B can be adjusted to the above range, for example, by adjusting the ratio between a filler mentioned later and a structure represented by RSiO_{3/2} or the amount of an organic functional group.

A large total occupancy maximum value or a cumulative total occupancy present on the high-log domain area ratio side means a structure with fewer voids or a structure having a wide dense domain with concentrated and continuing voids. A cumulative total occupancy on the low-log domain area ratio side means a structure with more voids and with uniformly distributed voids.

A large distribution of total occupancies against log domain area ratios means a large mottle of a void distribution. In the sensor of the present embodiment, fewer voids are preferred from the viewpoint of sensitivity, adhesiveness, and humidity resistance.

From the above viewpoint, it is preferred that at least one of the log domain area ratio A and the log domain area ratio B obtained by subjecting the acceptors in the third sensor and the fourth sensor to the measurement mentioned above should satisfy the corresponding range described above. From a similar viewpoint, it is preferred for the sensor of the present embodiment that both the log domain area ratio A and the log domain area ratio B should satisfy their respective ranges described above.

The acceptors in the first sensor and the second sensor preferably comprise an organic-inorganic hybrid. The organic-inorganic hybrid for the first sensor and the second sensor is preferably a structure represented by RSiO_{3/2} wherein the R represents an organic functional group, from the viewpoint of sensitivity and stability in a humid environment. The content of the organic-inorganic hybrid in the acceptors in the first sensor and the second sensor is not particularly limited and is preferably 50% by mass or more.

The acceptors in the third sensor and the fourth sensor comprise an organic-inorganic hybrid. The organic-inorganic hybrid is preferably a structure represented by RSiO_{3/2} wherein the R represents an organic functional group, from the viewpoint of sensitivity and stability in a humid environment.

In the third sensor, the void ratio as a volume fraction of the acceptor is 10% or less from the viewpoint of sensitivity and stability in a humid environment. The void ratio is more preferably 5% or less from the viewpoint of further improving sensitivity, further preferably 3% or less from the viewpoint of suppressing reduction in sensitivity in a humid environment. The void ratio can be adjusted to the above range, for example, by adjusting the ratio between a filler and the structure represented by RSiO_{3/2}. The void ratio is a value measured by a method described in the section of Examples of the present specification, or a method understood as being equivalent thereto by those skilled in the art.

From the same viewpoint as above, the void ratios of the acceptors in the first sensor, the second sensor and the fourth sensor are preferably 10% or less, more preferably 5% or more, further preferably 3% or less.

The acceptor in the fourth sensor comprises a first component having a weight-average molecular weight of 800 to 30000, and a second component having a weight-average molecular weight of 30000 to 2000000, and in a chromatogram obtained by measuring the acceptor by gel permeation chromatography (GPC), a peak area of the second component is 0.01% to 55% with respect to a total peak area of the first component and the second component, from the viewpoint of improving sensor sensitivity. For the GPC, specifically, a weight-average molecular weight based on polystyrene is measured with a RI detector and, more specifically, can be measured by a method described in Examples mentioned later.

The first component is a low-molecular-weight component, and its weight-average molecular weight is 800 or larger and 30000 or smaller, preferably 900 or larger and 15000 or smaller, from the viewpoint of coatability for forming the acceptor by inkjet coating, more preferably 1000 or larger and 8000 or smaller from the viewpoint of synthesis reproducibility. The first component is typically preferably derived from, but not limited to, the organic-inorganic hybrid.

The second component is a high-molecular-weight component, and its weight-average molecular weight is larger than 30000 and 2000000 or smaller, preferably 40000 or larger and 1900000 or smaller, from the viewpoint of further improving sensitivity, more preferably 50000 or larger and 1800000 or smaller from the viewpoint of coatability for forming the acceptor by inkjet coating. The second component is typically preferably derived from, but not limited to, a filler mentioned later. As a matter of course, the second component may contain a component derived from a condensate of a silane coupling agent such as tetraethoxysilane.

The ratio between the first component and the second component can be evaluated from the ratio of the peak area of the second component to the total peak area of the first component and the second component in a chromatogram obtained by GPC measurement. The peak area of the second component with respect to the total peak area of the first component and the second component is 0.01% to 55% from the viewpoint of improving sensor sensitivity, preferably 0.05% to 50% from the viewpoint of coatability for forming the acceptor by inkjet coating, more preferably 0.1% to 45% from the viewpoint of humidity resistance.

The weight-average molecular weights of the first component and the second component, and the ratio of the peak area can be adjusted to the above ranges, for example, by using a preferred organic-inorganic hybrid and filler mentioned later, or adjusting the ratio of the structure represented by RSiO_{3/2} or the amount of an organic functional group.

From the same viewpoint as above, preferably, the acceptors in the first sensor, the second sensor and the third sensor comprise a first component having a weight-average molecular weight of 800 to 30000, and a second component having a weight-average molecular weight of 30000 to 2000000, and in a chromatogram obtained by measuring the acceptor by gel permeation chromatography (GPC), a peak area of the second component is 0.01% to 55% with respect to a total peak area of the first component and the second component.

When the sensor of the present embodiment satisfies the requirement for one sensor selected from the group consisting of the first sensor, the second sensor, the third sensor and the fourth sensor, the sensor of the present embodiment tends to also satisfy the requirements for the other sensors. However, the sensor of the present embodiment can high-sensitively measure a highly hydrophilic gas such as ethanol or aldehyde even in a humid state and can further continuously measure targets without deteriorating the acceptor even after reflow treatment and after a long period of use, as long as the sensor corresponds to at least one of the first sensor, the second sensor, the third sensor and the fourth sensor. Specifically, the sensor of the present embodiment may not satisfy the requirement for any of the first sensor, the second sensor, the third sensor and the fourth sensor. In this respect, the sensor of the present embodiment can high-sensitively measure a highly hydrophilic gas such as ethanol or aldehyde even in a humid state and can further continuously measure targets without deteriorating the acceptor even after reflow treatment and after a long period of use, but not limited to, for example, (i) as long as the sensor does not correspond to the fourth sensor but corresponds to at least one of the first sensor, the second sensor and the third sensor, (ii) as long as the sensor does not correspond to the first sensor but corresponds to at least one of the second sensor, the third sensor and the fourth sensor, and (iii) as long as the sensor does not correspond to the second sensor but corresponds to at least one of the first sensor, the third sensor and the fourth sensor.

In the sensor of the present embodiment, the organic functional group preferably contains one or more types of aromatic rings. In the case of containing an aromatic ring, humidity resistance tends to be further improved. The aromatic ring can be appropriately selected in consideration of the purpose of the sensor, etc. and therefore, is not particularly limited. Examples thereof include aromatic hydrocarbon groups such as a phenyl group, a naphthyl group, a p-tolyl group, and a biphenyl group, substituted aromatic hydrocarbon groups such as a 4-chlorophenyl group, a 4-fluorophenyl group, a 4-bromophenyl group, a 4-iodophenyl group, a 4-methoxyphenyl group, a 4-aminophenyl group, and a pentafluorophenyl group, heterocyclic hydrocarbon groups such as a 3-furyl group, a 3-thienyl group, a 2-pyridyl group, a 3-pyridyl group, and a 4-pyridyl group, and metallocene such as a ferrocenyl group. The organic functional group in the sensor of the present embodiment may contain one singly of these aromatic rings or may contain two or more in combination of these aromatic rings.

In the organic-inorganic hybrid, a structure serving as a spacer, such as an alkyl group (having 1 to 18 carbon atoms), an ether group, a carbonyl group, a carboxyl group, an amide group, a urethane bond, a urea bond, an imide group, or an imine group may be sandwiched between the aromatic ring and the silicon atom. From the viewpoint of responsiveness sensitivity, it is preferred that the aromatic ring should be bonded directly to the Si atom in the organic-inorganic hybrid without such a spacer sandwiched.

The organic functional group in the sensor of the present embodiment preferably has a copolymer structure of the aromatic ring and a non-aromatic ring, from the viewpoint of achieving both humidity resistance and selectivity to various gas molecules (substances to be detected). Also, the organic functional group in the sensor of the present embodiment preferably comprises a functional group containing one or more atoms selected from the group consisting of a nitrogen atom, an oxygen atom, a sulfur atom, a halogen atom (e.g., fluorine, chlorine, bromine, and iodine atoms), a boron atom, and a phosphorus atom, from the viewpoint of responsiveness to highly polar gas or odor molecules. The organic functional group preferably comprises a functional group containing one or more atoms selected from the group consisting of a nitrogen atom, an oxygen atom, a sulfur atom, and a fluorine atom among those described above, from the viewpoint of stability.

Selectivity for a gas molecule can be easily changed from a molecular backbone or a copolymerization ratio. Examples of the measurable gas include oxidation-reduction gases, combustible gases, water vapor, and volatile organic compound (VOC) gases, more specifically, hydrocarbon, nitrogen oxides, sulfur oxides, carbon monoxide, carbon dioxide, hydrogen, oxygen, alcohols, amine, aldehyde, unsaturated aldehyde, ketone, ester, halomethane, volatile aromatic compounds, and volatile heterocyclic aromatic compounds. Examples of the hydrocarbon include methane, ethane, propane, butane, isobutane, and ethylene. Examples of the alcohol include methanol, ethanol, isopropanol, and higher alcohols. Examples of the amine include ammonia, dimethylamine, trimethylamine, and triethylamine. Examples of the aldehyde include formaldehyde and acetaldehyde. Examples of the unsaturated aldehyde include octenal, nonenal, and hexenal. Examples of the ketone include acetone. Examples of the halomethane include chloroform and dichloromethane. Examples of the volatile aromatic compound include benzene, toluene, xylene, and naphthalene. Examples of the volatile heterocyclic aromatic compound include indole and skatole.

Specific examples of the organic functional group in the sensor of the present embodiment include, but are not limited to, linear or branched hydrocarbon groups optionally having a substituent such as a halogen atom (e.g., a 3-chloropropyl group and linear or branched C₁₋₈ alkyl groups such as an octyl group), alicyclic hydrocarbon groups optionally having a substituent such as a halogen atom (e.g., C₅₋₆ cycloalkyl groups such as a cyclohexyl group), aromatic hydrocarbon groups optionally having a substituent such as a halogen atom (e.g., a phenyl group and a pentafluorophenyl group), a vinyl group, an allyl group, a (meth)acrylic group, (meth)acryloyloxyalkyl groups (e.g., (meth)acryloyloxy-C₁₋₃ alkyl groups such as a (meth)acryloyloxypropyl group), an epoxy group, glycidyloxyalkyl groups (e.g., glycidyloxy-C₁₋₃ alkyl groups such as a 3-glycidyloxypropyl group), (epoxycycloalkyl)alkyl groups (e.g., a 2-(3,4-epoxycyclohexyl)ethyl group), a hydroxy group, an ether group, a carbonyl group, a carboxyl group, an ester group, a urethane group, a urea group, a mercapto group, mercaptoalkyl groups (e.g., mercapto-C₁₋₃ alkyl groups such as a mercaptopropyl group), a sulfide group, amino groups (e.g., a primary amino group, a secondary amino group, and a tertiary amino group), aminoalkyl groups (e.g., amino-C₁₋₃ alkyl groups such as an aminopropyl group), (aminoalkylamino)alkyl groups (e.g., a 3-(2-aminoethylamino)propyl group), an imidazolyl group, imidazolylalkyl groups (e.g., a 3-(2-imidazolin-1-yl)propyl group), alkylaminoalkyl groups (e.g., a 3-(dimethylamino)propyl group), an amide group, a heterocyclic aromatic group, an oxime group, an imide group, an imine group, a halogen group, a nitrile group, a phosphone group, and a phosphoric acid group. Among them, the organic functional group is preferably one or more groups selected from the group consisting of a linear or branched hydrocarbon group optionally having a substituent, an alicyclic hydrocarbon group optionally having a substituent, an aromatic hydrocarbon group optionally having a substituent, a vinyl group, an allyl group, a (meth)acryloyloxyalkyl group, a glycidyloxyalkyl group, an (epoxycycloalkyl)alkyl group, a mercaptoalkyl group, an aminoalkyl group, an (aminoalkylamino)alkyl group, an imidazolylalkyl group, an alkylaminoalkyl group, and a hydroxy group, from the viewpoint of availability. One of these functional groups may be used singly, or two or more thereof may be used in combination.

The organic functional group is preferably an organic functional group derived from a silane coupling agent. Examples of the silane coupling agent include hydrolyzable metal oxides such as alkoxysilane, acetoxysilane and chlorosilane. Among them, the hydrolyzable metal oxide is preferably alkoxysilane because of handleability and the versatility of the functional group, more preferably trifunctional alkoxysilane from the viewpoint of reactivity.

The trifunctional alkoxysilane may be copolymerized with mono-, di- or tetrafunctional alkoxysilane or the like and is preferably copolymerized with tetrafunctional alkoxysilane from the viewpoint of enhancing the Young's modulus of the organic-inorganic hybrid for higher sensitivity.

A silane coupling agent having a methoxy group, an ethoxy group, an isopropoxy group, or a butoxy group as an alkoxy group can be used as the trifunctional alkoxysilane.

Examples of the commercially available aromatic silane coupling agent include, but are not limited to, phenyltrialkoxysilane, nitrobenzeneamidetrialkoxysilane, benzyltrialkoxysilane, 4-chlorophenyltrialkoxysilane, phenylaminopropyltrialkoxysilane, 4-aminophenyltrialkoxysilane, naphthyltrialkoxysilane, 4-methoxytrialkoxysilane, pentafluorophenyltrialkoxysilane, ferrocenyltrialkoxysilane, biphenyltrialkoxysilane, 3-furyltrialkoxysilane, 3-thienyltrialkoxysilane, 2-pyridyltrialkoxysilane, 3-pyridyltrialkoxysilane, and 4-pyridyltrialkoxysilane.

Examples of the commercially available non-aromatic silane coupling agent include, but are not limited to, 3-(2-aminoethylamino)propyltrialkoxysilane, 3-mercaptopropyltrialkoxysilane, vinyltrialkoxysilane, cyclohexyltrialkoxysilane, 3-glycidyloxypropyltrialkoxysilane, 2-(3,4-epoxycyclohexyl)ethyltrialkoxysilane, 3-(dimethylamino)propyltrialkoxysilane, 3-(2-imidazolin-1-yl)propyltrialkoxysilane, 3-aminopropyltrialkoxysilane, 3-(methacryloyloxy)propyltrialkoxysilane, 3-(acryloyloxy)propyltrialkoxysilane, allyltrialkoxysilane, methyltrialkoxysilane, ethyltrialkoxysilane, propyltrialkoxysilane, butylalkoxysilane, hexyltrialkoxysilane, octyltrialkoxysilane, decyltrialkoxysilane, dodecyltrialkoxysilane, octadecyltrialkoxysilane, 3-ureidopropyltrialkoxysilane, cyclohexylaminopropyltrialkoxysilane, hexafluorophenyltrialkoxysilane, 3-chloropropyltrialkoxysilane, 3-bromopropyltrialkoxysilane, 3-piperazinopropyltrialkoxysilane, 3-morpholinopropyltrialkoxysilane, 3-allylaminopropyltrialkoxysilane, norbornyltrialkoxysilane, and piperidinopropyltrialkoxysilane.

In the case of copolymerizing two or more types of silane coupling agents, the ratio between the aromatic silane coupling agent and the non-aromatic silane coupling agent is preferably a molar ratio of 1:10 to 1:5, preferably 1:5 to 1:1 from the viewpoint of heat resistance, and preferably 1:1 to 10:1 from the viewpoint of the suppression of reduction in signal intensity under humidity.

The organic functional group in the sensor of the present embodiment can be identified by subjecting the acceptor on the sensor body to surface element analysis such as SEM-EDX, XPS, or ATR.

### [Filler]

The acceptor according to the present embodiment preferably further comprises a filler. Use of the filler can improve the Young's modulus of the organic-inorganic hybrid and enhance sensitivity. The filler in the sensor of the present embodiment is not particularly limited and can be selected from various fillers known in the art. Specific examples thereof can include, but are not limited to, one or more fillers selected from spherical particles such as polymer (e.g., polystyrene, polymethyl methacrylate, and polyphenylene oxide) beads and acrylic latex, metal nitrides such as silicon nitride, metal oxides such as silica, zirconia, titania, zinc oxide, aluminum oxide, and tin oxide, composite metal compounds such as barium titanate, strontium titanate, and ITO, inorganic metal fillers such as gold, silver, copper, palladium, platinum, iron, and aluminum, and carbon materials such as carbon nanotubes, graphene, carbon black, carbon dots, and nanocarbon. The shape of the filler in the sensor of the present embodiment is not particularly limited, and a unique texture and/or structure such as a spherical shape, a beaded shape, a chain-like shape, a hollow shape, an oval spherical shape, a fibrous shape, a rod-like, a tubular shape, a needle-like shape, or a plate may be formed.

The filler in the sensor of the present embodiment is preferably an inorganic filler from the viewpoint of dispersibility and coatability. From the same viewpoint as above, the filler in the sensor of the present embodiment is preferably a metal oxide such as silica, zirconia, titania, or alumina, or an inorganic metal filler such as gold, silver, copper, palladium, platinum, iron, or aluminum, more preferably a metal oxide such as silica or zirconia from the viewpoint of small influence of signal attenuation ascribable to moisture under humidity, further preferably silica from the viewpoint of economic performance. The present inventors have found that, particularly, use of silica more markedly improves the attenuation of response of general hydrophilic gases such as ethanol or acetaldehyde even in a humid state. This is presumably because, but not limited to, since a hydroxy group present in a state bonded to a silica backbone and a silicon atom is relatively hydrophobic as compared with a hydroxy group bonded to a titania backbone or a titanium atom in Patent Literature 1, the inhibition of adsorption of a highly hydrophilic water molecule may be suppressed.

The shape of the filler in the sensor of the present embodiment is not particularly limited and is preferably a spherical shape, a rod-like shape, a plate-like shape or a fibrous shape, or a combined shape of two or more types thereof, more preferably a spherical shape or a chain-like shape. In this context, the spherical shape means a true spherical shape as well as a substantially spherical shape including a spheroid and an oval. The filler in the sensor of the present embodiment is particularly preferably spherical shape or chain-like silica.

The size of the spherical filler is not particularly limited and is preferably an average primary particle size of 100 µm or smaller from the viewpoint of being able to maintain ink dispersibility, i.e., to improve inkjet coatability, more preferably 500 nm or smaller from the viewpoint of being able to improve sensitivity, further preferably 200 nm or smaller from the viewpoint of further reducing voids. In this context, the average primary particle size means a number-average value. The average primary particle size is a number-average value of 50 particles by a method using a scanning electron microscope described in the section of Examples of the present specification, or a method understood as being equivalent thereto by those skilled in the art.

The chain-like filler preferably has a continuous chain-like structure. Preferred examples thereof include a structure where spherical particles are connected continuously and straight, a structure where such particles are connected in a branched pattern, a structure where such particles are connected in a bended pattern, a structure where such particles are connected in a circular pattern (also referred to as a structure where such particles are connected in a "pearl neckless pattern"), and a structure where many threadlike structures are entangled (also referred to as a structure where such particles are connected in a "network pattern") without individually continuous spherical shapes observed.

The filler having a continuous chain-like structure is not easy to define by a primary particle size by generally hypothesizing its spherical shape. However, the chain width can be observed by observation under a transmission or scanning electron microscope. The chain width is 1 nm to 200 nm, preferably 1 nm to 100 nm from the viewpoint of increasing a specific surface area, more preferably 1 nm to 85 nm from the viewpoint of the sensitivity of gas responsiveness. The average primary particle size is a number-average value of 50 particles by a method using a scanning electron microscope described in the section of Examples of the present specification, or a method understood as being equivalent thereto by those skilled in the art, and is defined as a chain width.

The metal oxide as such a filler can be produced by a sol-gel method using a metal oxide precursor having a tetrafunctional hydrolyzable group of alkoxy, chloro, or the like, or a commercially available product can also be used. Examples of the metal oxide precursor having a tetrafunctional hydrolyzable group include tetraalkoxysilane, tetrachlorosilane, tetraalkoxy titanium, and tetraacetoxy zirconium. Such a tetrafunctional hydrolyzable metal inorganic oxide may be copolymerized with a trifunctional hydrolyzable metal oxide, and its shape can be controlled by adjusting the concentration, temperature, pH, or water content of the reaction system. For example, in the case of producing the filler by emulsion polymerization, it is possible that the structure represented by RSiO_{3/2} is obtained on the filler side. The filler having such a structure in the acceptor according to the present embodiment is treated as the organic-inorganic hybrid in the sensor of the present embodiment.

Examples of the commercially available product of the filler that may be used can include: LEVASIL series (manufactured by H.C. Starck GmbH), Quatron P L series (manufactured by Fuso Chemical Co., Ltd.), and OSCAL series (manufactured by Catalysts & Chemicals Industries Co., Ltd.); powdery silica particles, for example, Aerosil 130, Aerosil 300, Aerosil 380, Aerosil TT600, and Aerosil OX50 (all manufactured by Nippon Aerosil Co., Ltd.), Sildex H31, Sildex H32, Sildex H51, Sildex H52, Sildex H121, and Sildex H122 (all manufactured by AGC Inc.), E220A and E220 (both manufactured by Nippon Silica Kogyo K.K.), SYLYSIA 470 (manufactured by Fuji Silysia Chemical Ltd.), SG flake (manufactured by Nippon Sheet Glass Co., Ltd.), IPA-ST, IPA-ST-L, IPA-ST-ZL, ST-XS, ST-S, ST-30, ST-50T, ST-30L, ST-YL, ST-ZL, MP-1040, MP-2040, MP-4540M, ST-OXS, ST-OS, ST-O, ST-O-40, ST-OL, ST-OYL, ST-NXS, ST-NS, ST-N, ST-N-40, ST-CXS, ST-C, ST-CM, ST-AK, ST-AK-L, ST-AK-YL, OZ-S30K, SZ-S30K-AC, OZ-S30M, Celnax CX-S505M, IPA-ST-UP, MEK-ST-UP, ST-UP, ST-PS-S, ST-PS-M, ST-OUP, ST-PS-SO, ST-PS-MO, and ST-AK-PS-S (manufactured by Nissan Chemical Corp.), and SRD-K, SRD-M, SXR-CM, SZR-K, and SZR-M (manufactured by Sakai Chemical Industry Co., Ltd.); and powdery fillers, for example, Aerosil 130, Aerosil 300, Aerosil 380, Aerosil TT600, and Aerosil OX50 (all manufactured by Nippon Aerosil Co., Ltd.), Sildex H31, Sildex H32, Sildex H51, Sildex H52, Sildex H121, and Sildex H122 (all manufactured by AGC Inc.), E220A and E220 (both manufactured by Nippon Silica Kogyo K.K.), SYLYSIA 470 (manufactured by Fuji Silysia Chemical Ltd.), SG flake (manufactured by Nippon Sheet Glass Co., Ltd.). The surfaces of these fillers may be chemically modified for use.

### [Method for producing organic-inorganic hybrid]

The method for producing the organic-inorganic hybrid is not particularly limited, and the organic-inorganic hybrid is typically produced by use of a sol-gel method. Specifically, a hydrolyzable silane compound (including a silane coupling agent) can be treated by the sol-gel method under acidic or basic conditions in the presence of water to obtain a condensate of the silane coupling agent, i.e., an organic-inorganic hybrid having a structure represented by RSiO_{3/2}. The organic-inorganic hybrid according to the present embodiment used can have a basket-shaped, ladder-shaped, or random molecular backbone, or a mixture thereof may be used.

The silane coupling agent is more preferably hydrolyzed, condensed, and surface-modified in the presence of a catalyst from the viewpoint of being able to adjust the reaction rates of hydrolysis and condensation.

Examples of the type of the catalyst include acid catalysts and base catalysts. Examples of the acid catalyst include inorganic acids and organic acids. Examples of the inorganic acid include, but are not limited to, hydrochloric acid, nitric acid, sulfuric acid, hydrofluoric acid, phosphoric acid, and boric acid. Examples of the organic acid include, but are not limited to, acetic acid, propionic acid, butanoic acid, pentanoic acid, hexanoic acid, heptanoic acid, octanoic acid, nonanoic acid, decanoic acid, oxalic acid, maleic acid, methylmalonic acid, benzoic acid, p-aminobenzoic acid, p-toluenesulfonic acid, benzenesulfonic acid, trifluoroacetic acid, formic acid, malonic acid, sulfonic acid, phthalic acid, fumaric acid, citric acid, tartaric acid, citraconic acid, malic acid, and glutaric acid. Examples of the base catalyst include inorganic bases and organic bases. Examples of the inorganic base include, but are not limited to: alkali metal hydroxides such as lithium hydroxide, sodium hydroxide, potassium hydroxide, and cesium hydroxide; alkaline earth metal hydroxides such as calcium hydroxide; alkali or alkaline earth metal carbonates such as lithium carbonate, potassium carbonate, and sodium carbonate; and metal bicarbonates such as potassium bicarbonate and sodium bicarbonate. Examples of the organic base include, but are not limited to: ammonia water; trialkylamine such as triethylamine and ethyldiisopropylamine; N,N-dialkylaniline derivatives having 1 to 4 carbon atoms, such as N,N-dimethylaniline and N,N-diethylaniline; and pyridine derivatives optionally having an alkyl substituent having 1 to 4 carbon atoms, such as pyridine and 2,6-lutidine. These catalysts can be used each singly or as a mixture of two or more thereof.

From the viewpoint of reaction efficiency, it is preferred to add the catalyst in an amount that allows the pH of the reaction system to fall within the range of 0.01 to 6.0 or attains pH 8 to 14. It is more preferred for further enhancing efficiency to perform sol-gel reaction under basic conditions of pH 8 to 14.

The hydrolysis and condensation for producing the organic-inorganic hybrid may be performed in an organic solvent. Examples of the organic solvent that may be used in the condensation reaction include alcohols, ester, ketone, ether, aliphatic hydrocarbon compounds, aromatic hydrocarbon compounds, and amide compounds.

Examples of the alcohol include, but are not limited to: monohydric alcohols such as methyl alcohol, ethyl alcohol, propyl alcohol, and butyl alcohol; polyhydric alcohols such as ethylene glycol, diethylene glycol, propylene glycol, glycerin, trimethylolpropane, and hexanetriol; and monoethers of polyhydric alcohols such as ethylene glycol monomethyl ether, ethylene glycol monoethyl ether, ethylene glycol monopropyl ether, ethylene glycol monobutyl ether, diethylene glycol monomethyl ether, diethylene glycol monoethyl ether, diethylene glycol monopropyl ether, diethylene glycol monobutyl ether, propylene glycol monomethyl ether, propylene glycol monoethyl ether, propylene glycol monopropyl ether, and propylene glycol monobutyl ether.

Examples of the ester include, but are not limited to, methyl acetate, ethyl acetate, butyl acetate, and γ-butyrolactone. Examples of the ketone include, but are not limited to, acetone, methyl ethyl ketone, and methyl isoamyl ketone.

Examples of the ether include the monoethers of polyhydric alcohols described above as well as: polyhydric alcohol ethers in which all the hydroxy groups of a polyhydric alcohol are alkyl-etherified, such as ethylene glycol dimethyl ether, ethylene glycol diethyl ether, ethylene glycol dipropyl ether, ethylene glycol dibutyl ether, propylene glycol dimethyl ether, propylene glycol diethyl ether, propylene glycol dibutyl ether, diethylene glycol dimethyl ether, diethylene glycol methyl ethyl ether, and diethylene glycol diethyl ether; tetrahydrofuran; 1,4-dioxane; and anisole.

Examples of the aliphatic hydrocarbon compound include, but are not limited to, hexane, heptane, octane, nonane, and decane.

Examples of the aromatic hydrocarbon compound include, but are not limited to, benzene, toluene, and xylene.

Examples of the amide compound include, but are not limited to, dimethylformamide, dimethylacetamide, and N-methylpyrrolidone.

Among these solvents, an alcohol such as methanol, ethanol, isopropanol, or butanol; ketone such as acetone, methyl ethyl ketone, or methyl isobutyl ketone; ether such as ethylene glycol monomethyl ether, diethylene glycol monobutyl ether, propylene glycol monomethyl ether, or propylene glycol monoethyl ether; or an amide compound such as dimethylformamide, dimethylacetamide, or N-methylpyrrolidone is preferred from the viewpoint of being easily mixed with water. These solvents may be used each singly, or a plurality of solvents may be used in combination.

In the case of blending a filler into the organic-inorganic hybrid, the organic-inorganic hybrid may be produced in the presence of the filler, or the filler may be blended into the produced organic-inorganic hybrid. The condensate of the silane coupling agent may be dissolved in a commercially available dispersed filler, or the filler and the silane coupling agent may be dispersed with a ball mill, a jet mill, or the like. Alkoxysilane and the filler can be subjected to condensation reaction at the same time for production. A production method of condensing alkoxysilane and the filler at the same time is more preferred from the viewpoint of enhancing the solvent dispersibility of the filler and enhancing inkjet coatability. In the case of treating a colloidal filler by the sol-gel method in the presence of a silane coupling agent, a condensate of the silane coupling agent modifies the surface of the filler while the condensate is arranged so as to fill a gap. Thus, the amount of pores can be adjusted by arbitrarily adjusting the amount of the alkoxysilane. When a hydroxy group is present in the filler surface, the surface hydroxy group and a hydroxy group formed by the hydrolysis of the alkoxysilane form an ether bond through interaction and condensation reaction. Therefore, a more stable acceptor can be obtained because the compatibility between the filler and the condensate can be enhanced.

The surface modification of the filler with the organic functional group is performed by surface modification in a state dispersed in a dispersion medium. Specifically, the organic-inorganic hybrid is produced, for example, by the step of adding the silane coupling agent to a homogeneous dispersion of a metal oxide containing an alcohol and water in the presence of a catalyst.

The organic-inorganic hybrid may be recovered by filtering off precipitates and gels by centrifugation and filtration, and is isolated by removing a volatile solvent with an evaporate or the like.

The introduction of the organic functional group can be confirmed by FT-IR or elemental analysis. This can also be confirmed by thermal weight reduction measurement. The thermal weight reduction rate of combined organic matter of the inorganic filler (i) and the organic functional group (ii) from 150°C to 1000°C in order to remove the influence of adsorbed water is preferably 3% to 75% from the viewpoint of sensitivity, more preferably 5% to 65% from the viewpoint of humidity resistance, further preferably 5% to 60% from the viewpoint of heat stability. Specifically, the amount of the organic functional group in the organic-inorganic hybrid in the sensor of the present embodiment is not particularly limited and is preferably 75% by mass or less, more preferably 65% by mass or less, from the viewpoint of the balance between responsiveness to a substance to be detected and the stability of the acceptor. Alternatively, the acceptor according to the present embodiment may comprise no filler. In this case, the requirements for the first sensor, the second sensor, the third sensor and the fourth sensor tend to be satisfied by increasing the amount of the organic functional group. From such a viewpoint, the amount of the organic functional group in the organic-inorganic hybrid in the sensor of the present embodiment is preferably 3% by mass or more and 75% by mass or less, more preferably 5% by mass or more and 65% by mass or less.

The acceptor according to the present embodiment preferably has a membrane shape because measurement accuracy is stable. As for its film thickness, the acceptor according to the present embodiment may have a shape where the organic-inorganic hybrid has an even film thickness distribution, or may have a coffee-ring shape where film thicknesses are distributed such that the end portion is thick. The membrane having a coffee-ring form is preferred because this membrane is free from shape deterioration even when exposed to vapor with a high concentration of VOC, is excellent in structural stability, and has high sensitivity as compared with a membrane structure consisting of a flat structure. This is presumably because, but not limited to, when the sensor is a surface stress sensor, a coffee-ring membrane disposed on the surface stress sensor may have a more membrane volume near piezoelectricity and therefore more efficiently propagate stress ascribable to deformation thereof to a piezoelectric element; thus higher sensitivity may be achieved. For the coffee-ring type, a cross-sectional profile between two points on the diameter of a membrane formed in a round shape is obtained, and the film thickness of a central portion from the cross-sectional profile with respect to the largest film thickness is preferably 80% or less from the viewpoint of sensitivity reproducibility, more preferably 60% or less from the viewpoint of sensitivity, further preferably 40% or less from the viewpoint of the maximization of response sensitivity. In the case of a coffee-ring shape, the film thickness of each of the thinnest portion in the central portion of the membrane, and the thickest portion in the end portion is usually preferably 10 nm or larger and 50 µm or smaller. The film thickness is preferably 10 nm or larger and 30 µm or smaller from the viewpoint of shortening the time required for drying at the time of production and enhancing productivity, more preferably 10 nm or larger and 20 µm or smaller from the viewpoint of enhancing sensitivity.

The largest thickness of the acceptor according to the present embodiment is preferably 30 µm or smaller. The film thickness and the largest thickness can be measured by a method described in Examples mentioned later.

In the case of using a surface stress sensor having a sensor body covered with the acceptor according to the present embodiment as an olfactory sensor, it is preferred that the organic functional group should interact with a gas molecule in a gas phase so that stress is generated by the adsorption or internal diffusion of the gas molecule by the acceptor, thereby detecting the gas.

### [Surface stress (MSS) sensor]

The sensor of the present embodiment can be applied to, for example, a surface stress sensor and, more specifically, can be used as a membrane-type surface stress (MSS) sensor. In this case, a substance to be detected is adsorbed onto the acceptor covering at least a portion of the surface of the sensor body so that the body detects change in stress caused in the acceptor and outputs signals. The surface stress sensor is preferably a four-point fixed piezoelectric element-type surface stress sensor.

### [Method for producing sensor]

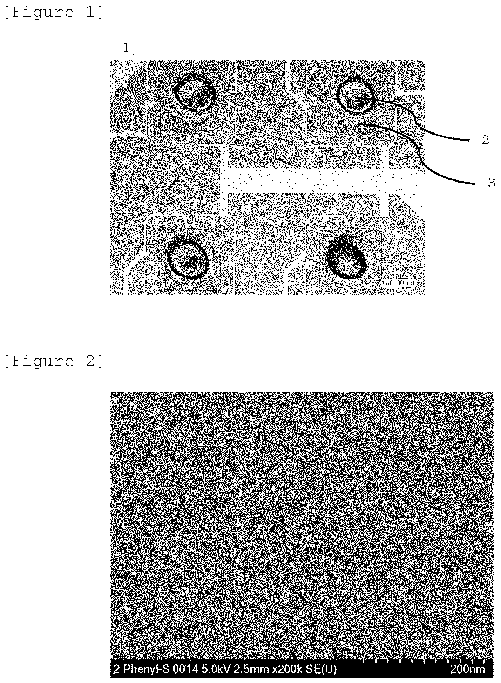

The method for producing the sensor of the present embodiment is not particularly limited, and the sensor of the present embodiment can be produced by various methods for forming an acceptor on a sensor body. The acceptor according to the present embodiment is produced, for example, by dispersing an organic-inorganic hybrid in an organic solvent to prepare a coating liquid, and coating a sensor body with the coating liquid. An inkjet apparatus can be suitably used in coating a sensor chip with the coating liquid. The inkjet apparatus injects liquid droplets of the coating liquid so that the sensor is coated with the liquid droplets to form a membrane of the acceptor. An example in which the acceptor is formed on a chip is shown in Figure 1. Sensor 1 is configured to have chip 3 corresponding to the sensor body, and acceptor 2 formed on its surface. In the example shown in Figure 1, such a configuration is provided at four locations.

Examples of the approach for covering the surface of the sensor body (e.g., a MSS sensor body) with the organic-inorganic hybrid include, but are not particularly limited to, covering methods using dip coating, spray coating, spin coating, inkjet spotting, casting, or a doctor blade.

For coating a 300 µmφ membrane, an inkjet or microjet system is preferred, and a microjet coating system is more preferred from the viewpoint of productivity.

The acceptor according to the present embodiment can be produced, for example, by dispersing the organic-inorganic hybrid in an organic solvent, and coating a MSS sensor chip with the dispersion. The organic-inorganic hybrid is dispersed at a concentration of 0.1 g/L to 50 g/L in an organic solvent to prepare a coating liquid. The concentration is preferably 0.5 to 50 g/L from the viewpoint of productivity, more preferably 0.5 to 15 g/L from the viewpoint of suppressing nozzle clogging.

The organic solvent that disperses therein the organic-inorganic hybrid can be any solvent capable of dispersion and preferably has a boiling point of 80°C to 250°C. The boiling point is preferably 100°C to 250°C for suppressing reduction in productivity caused by nozzle clogging, more preferably 100°C to 200°C for shortening the time required for drying and enhancing productivity.

Examples of the organic solvent include, but are not limited to, alcohol solvents, ester solvents, ketone solvents, ether solvents, aliphatic hydrocarbon solvents, aromatic hydrocarbon solvents, and amide solvents.

Examples of the alcohol solvent include, but are not limited to: monohydric alcohols such as methyl alcohol, ethyl alcohol, propyl alcohol, and butyl alcohol; polyhydric alcohols such as ethylene glycol, diethylene glycol, propylene glycol, glycerin, trimethylolpropane, and hexanetriol; and monoethers of polyhydric alcohols such as ethylene glycol monomethyl ether, ethylene glycol monoethyl ether, ethylene glycol monopropyl ether, ethylene glycol monobutyl ether, diethylene glycol monomethyl ether, diethylene glycol monoethyl ether, diethylene glycol monopropyl ether, diethylene glycol monobutyl ether, propylene glycol monomethyl ether, propylene glycol monoethyl ether, propylene glycol monopropyl ether, and propylene glycol monobutyl ether.

Examples of the ester solvent include methyl acetate, ethyl acetate, butyl acetate, and γ-butyrolactone.

Examples of the ketone solvent include, but are not limited to, acetone, methyl ethyl ketone, and methyl isoamyl ketone.

Examples of the ether solvent include, but are not limited to: polyhydric alcohol ethers in which all the hydroxy groups of a polyhydric alcohol are alkyl-etherified, such as ethylene glycol dimethyl ether, ethylene glycol diethyl ether, ethylene glycol dipropyl ether, ethylene glycol dibutyl ether, propylene glycol dimethyl ether, propylene glycol diethyl ether, propylene glycol dibutyl ether, diethylene glycol dimethyl ether, diethylene glycol methyl ethyl ether, and diethylene glycol diethyl ether; tetrahydrofuran; 1,4-dioxane; and anisole.

Examples of the aliphatic hydrocarbon solvent include, but are not limited to, hexane, heptane, octane, nonane, and decane.

Examples of the aromatic hydrocarbon solvent include, but are not limited to, benzene, toluene, and xylene.

Examples of the amide solvent include, but are not limited to, N,N-dimethylformamide, N,N-dimethylacetamide, and N-methylpyrrolidone.

One of these organic solvents may be used singly, or two or more thereof may be used in combination.

Among these organic solvents, N,N-dimethylformamide or propylene glycol monomethyl ether is preferred because of excellent safety and solubility.

The acceptor according to the present embodiment may contain arbitrary additives such as a particle, an ionic compound, a resin, and a low-molecular compound, in addition to the organic-inorganic hybrid. Such an additive can be blended into, for example, the coating liquid, with which the surface of a sensor body is then coated to obtain a sensor having an acceptor containing the additive.

### Examples

Hereinafter, the present embodiment will be specifically described with reference to Examples and Comparative Examples. However, the present embodiment is not limited by these examples.

Various physical properties of a MSS sensor of each of Examples and Comparative Examples were evaluated according to the following (1) to (8).

### (1) Measurement of void ratio of acceptor

A sample having a cross section formed by singulation was fixed onto a processing table, then processed by FIB milling described below, and observed under a scanning electron microscope (SEM). The SEM observation conditions were as described below.
(Processing conditions: FIB milling)
Apparatus: Helios 650
Crude processing: carried out after protective membrane formation with W
Accelerating voltage: 30 kV
Current value: 9.4 nA, 2.4 nA, and 0.7 nA changed in this order
(Finish processing)
Accelerating voltage: 30 kV
Current value: 0.24 nA
(SEM observation conditions)
Apparatus: Helios 650 (manufactured by Thermo Fisher Scientific Inc.)
Accelerating voltage: 2 kV, 0.1 or 0.2 nA
Observation image: reflection electron image (composition image)
Slope: 52°

A micro concave-convex-shaped layer was cut out of an image obtained by the SEM observation of the cross section, and voids present in an organic-inorganic hybrid were further extracted. For the extraction, binarization treatment was performed with image analysis software through the use of the fact that: the peripheries of the voids in the obtained image had a bright contrast owing to edge effects; and the void themselves had a dark contrast. This binarization treatment was performed such that the micro concave-convex-shaped layer was indicated by a white color and the voids, a substrate and the background were indicated by a black color. A site that was not able to be followed by the binarization treatment was complemented by manual identification. In the obtained image of the extracted voids, the ratio of the area of portions corresponding to the voids to the area of portions corresponding to the micro concave-convex-shaped layer and the voids was calculated as a void ratio in the micro concave-convex-shaped layer. This operation was performed for 3 fields of view, and a number-average value thereof was regarded as the void ratio.

### (2) Measurement of void parameter of acceptor

A cross section in the thickness direction of an acceptor on a sensor body was formed by singulation. This sample was fixed onto a processing table, then processed by FIB milling under conditions described below, and observed under SEM. The SEM observation conditions were as described below.
(Processing conditions: FIB milling)
Apparatus: Helios 650
Crude processing: carried out after protective membrane formation with W
Accelerating voltage: 30 kV
Current value: 9.4 nA, 2.4 nA, and 0.7 nA changed in this order
(Finish processing)
Accelerating voltage: 30 kV
Current value: 0.24 nA
(SEM observation conditions)
Apparatus: Helios 650 (manufactured by Thermo Fisher Scientific Inc.)
Accelerating voltage: 2 kV, 0.1 or 0.2 nA
Observation image: reflection electron image (composition image)
Slope: 52°
Magnification: 100000x

An acceptor layer was cut out of an image obtained by the SEM observation of the cross section, and voids present in the cross section of the acceptor were further extracted. For the extraction, noise was first removed from the image through a median filter using 10 neighbor pixels, then a threshold was defined on a domain basis of the image, and binarization treatment that indicated dense sites by a white color and voids by black color was performed by adaptive threshold processing, which is a method capable of binarization resistant to change in light source environment within images, through the use of the fact that: the peripheries of the voids in the obtained image had a bright contrast owing to edge effects; and the void themselves had a dark contrast. This threshold was set to 131 in terms of grayscale. The obtained image of the extracted voids was subjected to 3 × 3 filtering so as not to miss 1-pixel voids. After void dilation, quadtree spatial partitioning was carried out in order to discriminate between the void portions and dense portions of the cross section. The quadtree spatial partitioning was continued until the number of void pixels (black pixels) contained in each domain of the partitions became 1. A quadrangle group corresponding to the dense portions free from void pixels was classified on an area basis, and the log domain area ratio and the total occupancy were calculated as follows.

### (Log domain area ratio)

A value determined by dividing each area of the quadrangle group by the area of the image was regarded as a domain area ratio, and a common logarithm thereof was taken as the log domain area ratio.

### (Total occupancy)

A total area value of quadrangles having the same area in the quadrangle group was indicated by a ratio with the area of the image defined as 1, and the resulting value was regarded as the total occupancy.

### (Log domain area ratio A and log domain area ratio B)

The log domain area ratios thus obtained were plotted on the abscissa against the total occupancies thus obtained on the ordinate. Also, the log domain area ratios thus obtained were plotted on the abscissa against the cumulative total occupancies thus obtained on the ordinate. In the resulting distribution of the total occupancies against the log domain area ratios, log domain area ratio A which provided a total occupancy maximum, and log domain area ratio B which provided a cumulative total occupancy of 60% in an ascending order of the log domain area ratios, were determined. The measurement was carried out as to at least 3 points in each acceptor or as to at least 5 points if a distribution was seen in voids, and the largest values were determined as the log domain area ratio A and the log domain area ratio B.

### (3) Identification of organic functional group species in organic-inorganic hybrid

An acceptor on a sensor body was measured by microscopic ATR on the basis of conditions described below to identify an organic functional group in an organic-inorganic hybrid.

### (Measurement conditions)

Apparatus used: Cary 620 (manufactured by Agilent Technologies Japan Ltd.)
Measurement method: microscopic ATR (crystal: Ge) using slide-on ATR attachment
Detector: MCT (HgCeTe) infrared detection element Aperture size: approximately 100 µm
IR incident angle into ATR crystal: 30°
Resolution: 4 cm⁻¹
The number of scans: 64 scans

### (4) Thermal weight reduction rate (quantification of amount of organic functional group)

A powder sample of an acceptor scraped from a MSS sensor was dried at 100°C in the atmosphere, subsequently brought back to room temperature, and then accurately weighed. Then, organic components were removed by heating to 1000°C at a temperature increase rate of 10°C/min in the atmosphere using TGA to determine a thermal weight reduction rate. Since only the organic functional group was not decomposed by heating and the SiO_{3/2} moiety remained as silica, the thermal weight reduction rate was regarded as the amount of the organic functional group (% by mass).

### (5) Largest thickness of acceptor

An acceptor-uncoated portion and the largest-film thickness portion of the acceptor in a MSS sensor were observed under a confocal laser microscope (VK09700 Violet Laser (manufactured by Keyence Corp.)) to measure the largest thickness of the acceptor. Specifically, a cross-sectional profile between two points on the diameter of the acceptor formed in a substantially round shape was obtained, and the acceptor-uncoated portion and the largest-film thickness portion of the acceptor were identified from the cross-sectional profile. A difference therebetween was obtained to determine the largest thickness.

### (Observation conditions)

Objective lens: standard lens, 20.0×
Measurement area: surface
Measurement mode: surface shape
RPD: accuracy prioritized
Measurement quality: high resolution
Measurement pitch: 0.5 µm
Z measurement distance: 37.92 µm
Wide dynamic range: OFF
Brightness 1: 648
Neutral density filter: 100%
Optical zoom: 1×
Average number of times: once
Filter: OFF
White balance mode: one push
White balance R: 131
White balance B: 138
Received light quantity correction mode: γ correction
γ correction value: 0.45
γ offset: 0
Black-and-white inversion: OFF
Head type: VK-9700
Light quantity eccentricity correction: ON
Image curvature correction: ON
XY calibration: 688 nm/pixel
Z calibration: 1 nm/digit
Brilliance: 5
Contrast: 5
Brightness: 0

### (6) Resistance to influence of humidity

A sensor was charged with 100 ppm ethanol at 25°C for 10 minutes and purged with nitrogen alone for 10 minutes three repetitive times each, and signals from the third round were adopted. An attenuation rate (%) was determined according to 100 × ("Intensity of the signals from the third round measured under drying" - "Intensity of the signals from the third round measured under 30 RT%") / "Intensity of the signals from the third round measured under drying". A sample having a value of 20% or less was graded S; a sample having a value of 21% to 40% or less was graded as A; a sample having a value of 41% or more and less than 60% was graded B; and a sample having a value of 61% or more was graded C.

### (7) Physical or chemical stability

### (First round)

Saturated water vapor was used as a substance to be detected. A sensor was purged (whole flow rate was set to 30 sccm) with saturated water vapor together with 6 sccm nitrogen at 25°C for 10 minutes under dry conditions, and signal intensity was measured.

### (Second round)

Ethanol was used as a substance to be detected. A sensor was purged (whole flow rate was set to 30 sccm) with headspace gas of ethanol together with 6 sccm nitrogen at 25°C for 10 minutes under dry conditions, and signal intensity was measured.

### (Third round)

Toluene was used as a substance to be detected. A sensor was purged (whole flow rate was set to 30 sccm) with headspace gas of toluene together with 6 sccm nitrogen at 25°C for 10 minutes under dry conditions, and signal intensity was measured.

After measurement in each round, change in membrane shape was observed by observation under an optical microscope and evaluated as follows.
A ... No change was seen even after the third round.
B ... Although no change was seen after the second round, evident change in membrane shape was seen after the third round under a microscope at a 200× magnification.
C ... Evident change in membrane shape was seen after the second round under a microscope at a 200× magnification.

### (8) Heat resistance (reflow resistance)

After reflow at 250°C for 30 seconds, a sample in which peel, deformation, cracks, or the like was seen in an acceptor was graded C, and a sample in which no such defect was seen was graded A.

### (9) Filler particle size (diameter)

A number-average value of 50 filler particles was determined under the scanning electron microscope described above in (1). The largest size was regarded as a particle size for spherical particles, and a chain width was regarded as a particle size for chain-like particles. A number-average value of 50 particles each was calculated.

### (10) Molecular weight measurement by gel permeation chromatography (GPC)

An acceptor material was dissolved in N,N-dimethylformamide (DMF), and the solution was passed through a 5 µm PTFE syringe filter to prepare a sample. GPC measurement was performed using HLC-8420GPC manufactured by Tosoh Corp. Analysis conditions were as follows.

### <Analysis conditions>

Column: TSK gel SuperAWM-H × 2
Flow rate: 0.6 mL/min
Detector: RI detector with built-in HLC-8420GPC + UV-8420
Column temperature: 40°C
System temperature: 40°C
Eluent: 10 mM LiBr in DMF
Calibration curve conditions: standard polystyrene KIT-H (manufactured by Tosoh Corp.)

The analysis was conducted using a RI detector, and the weight-average molecular weight was calculated using EcoSEC Elite-WS software. The signal intensity ratios of a high weight-average molecular weight and a low weight-average molecular weight were calculated using signal intensity determined in the RI detector. In tables, numeric values obtained by rounding off the first component to the 100 and rounding off the second component to the 1000 were each described.

### <Example 1>

In a separable flask, 27.1 g of phenyltrimethoxysilane was added to 50 g of dispersion IPA-ST-UP of chain-like colloidal silica in 2-propanol (product of Nissan Chemical Corp.), 90 g of water, 10 g of 28% ammonia water, and 300 g of 2-propanol, and the mixture was reacted at 80°C for 4 hours. The reaction solution was cooled in air and then centrifuged at 6000 rpm for 10 minutes to isolate precipitates. The sample was thoroughly washed with ethanol and then dissolved at 10 mg/mL in DMF to obtain a coating liquid.

A portion of the coating liquid was used as a sample and dried at 100°C in the atmosphere as described above in (4), and its thermal weight reduction rate was measured.

The surface of a sensor body for a MSS sensor disposed on a hot plate of 80°C was coated with a portion of the coating liquid using an inkjet apparatus to obtain a MSS sensor equipped with an acceptor (sensitive membrane). In this coating, first, the surface was continuously coated with 300 pL each of two liquid droplets (600 pL) and dried for 1 second, and the same operation was repeated (a total of 30 times). Specifically, 18000 pL of the coating liquid was used. A typical appearance of the MSS sensor was the same as that shown in Figure 1.

The surface of the MSS sensor according to Example 1 was observed under SEM. The obtained image as to the microstructure of the central portion of the acceptor is shown in Figure 2. As seen from Figure 2, the acceptor was uniformly formed on the sensor body, at the central portion of the acceptor in the MSS sensor according to Example 1.

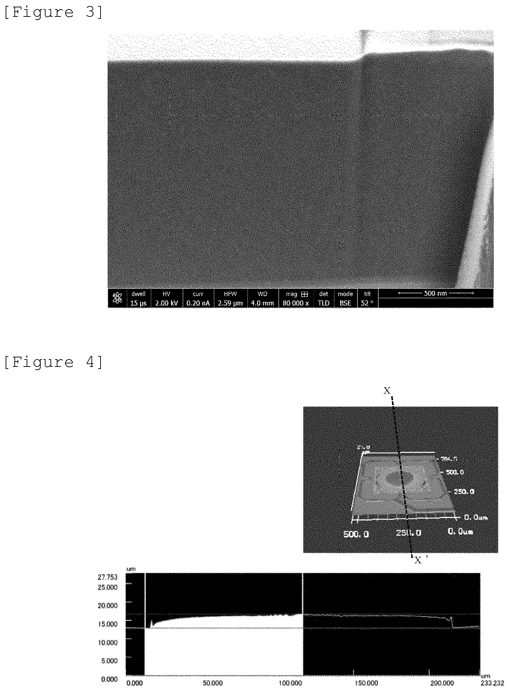

Subsequently, the cross section of the MSS sensor according to Example 1 was observed under SEM. The results are shown in Figure 3. The void ratio was calculated as described above in (1) on the basis of such a SEM image of the cross section.

The largest thickness of the MSS sensor according to Example 1 was measured on the basis of the method described above in (5). As a result, the upper image of Figure 4 was obtained. As seen from the upper image of Figure 4, a coffee-ring acceptor was formed on a 700 µm long × 500 µm wide sensor body. The film thickness was further analyzed. As a result, the film thickness distribution shown in the lower part of Figure 4 was obtained. The lower image of Figure 4 shows a cross-sectional profile between two points on the X-X' cross section in the upper image of Figure 4. The largest-film thickness portion and the acceptor-uncoated portion were identified on the basis of this profile, and the largest thickness was determined from a difference therebetween.

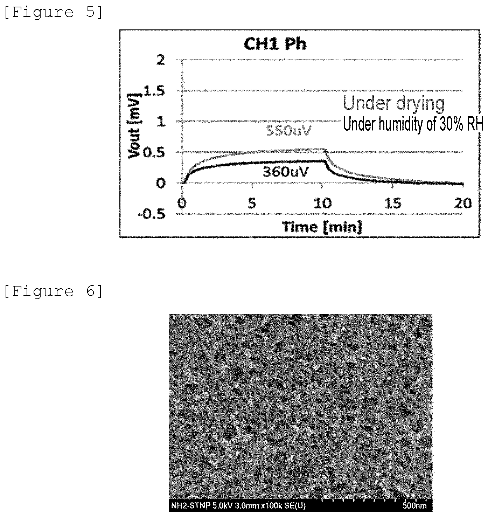

The resistance to the influence of humidity was studied on the basis of the method described above in (6). The results are shown in Figure 5. A maximum amplitude of 550 µV was obtained at a humidity of 0% (under drying), and a maximum amplitude of 360 µV was obtained at a humidity of 30%. The attenuation rate calculated by the method mentioned above was 40%. Therefore, this sample was graded A.

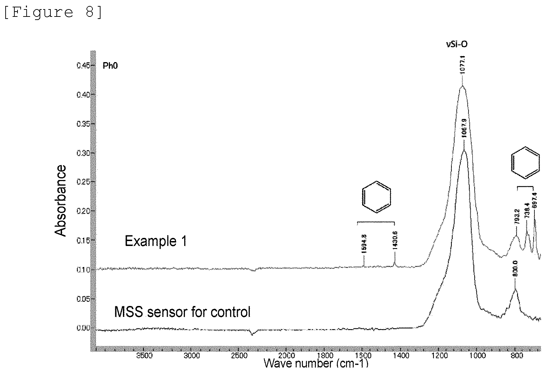

Aside from this, dispersion IPA-ST-UP of chain-like colloidal silica in 2-propanol (product of Nissan Chemical Corp.) was diluted into 10 mg/mL with DMF. A MSS surface disposed on a hot plate of 80°C was coated with 350 droplets in an amount of 300 pL per droplet using an inkjet apparatus to prepare a MSS sensor for a control. The organic functional group in the acceptor on the sensor body was identified as to this MSS sensor for a control and the MSS sensor of Example 1 by the method described above in (3). The results are shown in Figure 8. Signals derived from the introduced phenyl group were detected in the MSS sensor of Example 1. Also, the structure represented by RSiO_{3/2} was observed in Example 1, whereas no such structure was observed in the MSS sensor for a control. The organic functional group species was similarly identified in other Examples and Comparative Examples.

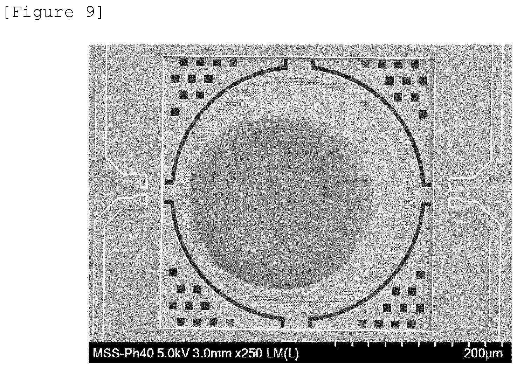

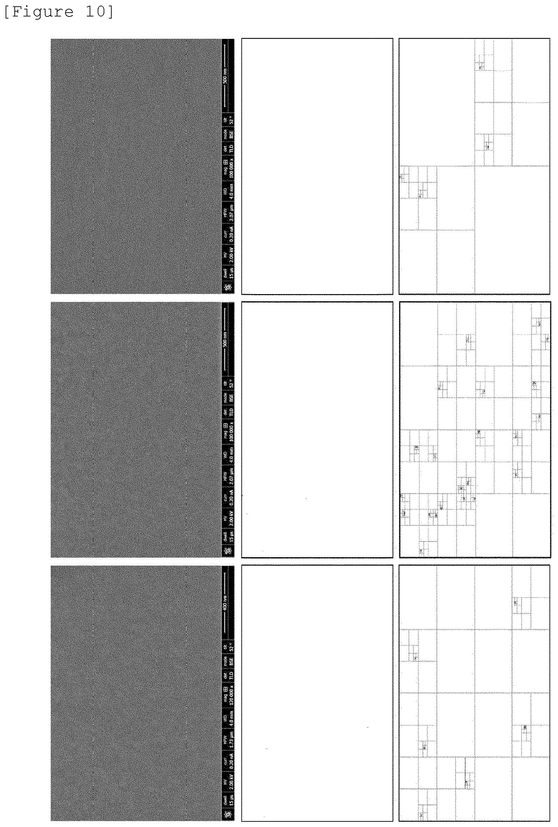

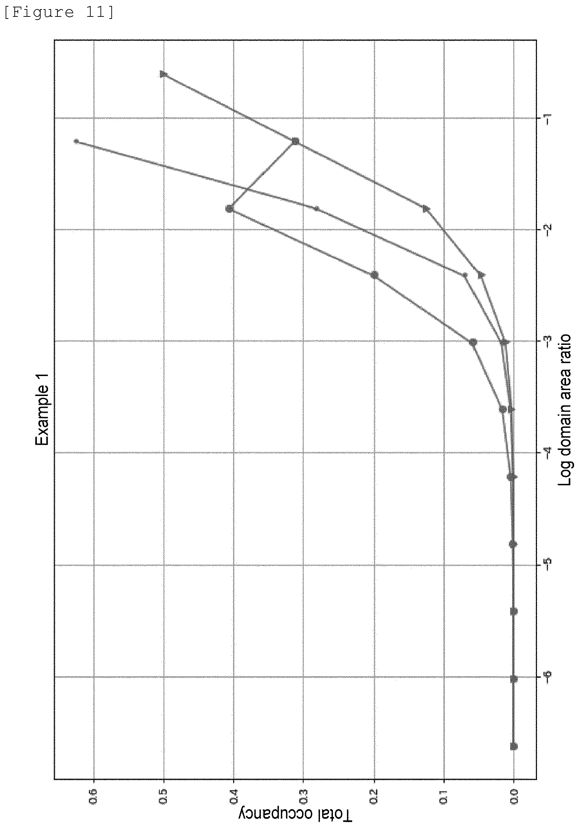

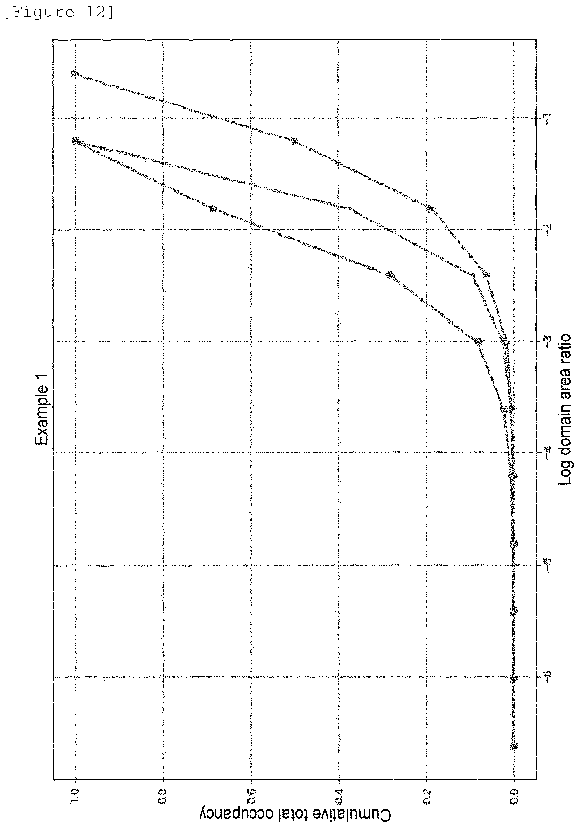

Subsequently, the surface of the acceptor in Example 1 was separately observed under SEM. The results are shown in Figure 9. SEM images of cross sections at arbitrary three points of the acceptor were obtained and binarized, followed by quadtree spatial partitioning by the method described above in (2) (Figure 10). After the quadtree spatial partitioning, total occupancies and cumulative total occupancies were plotted against log domain area ratios. As a result, the largest log area occupancy A and log area occupancy B of the three cross sections were -0.60 and -1.10, respectively (Figures 11 and 12). Thus, few voids were seen in the acceptor according to Example 1.

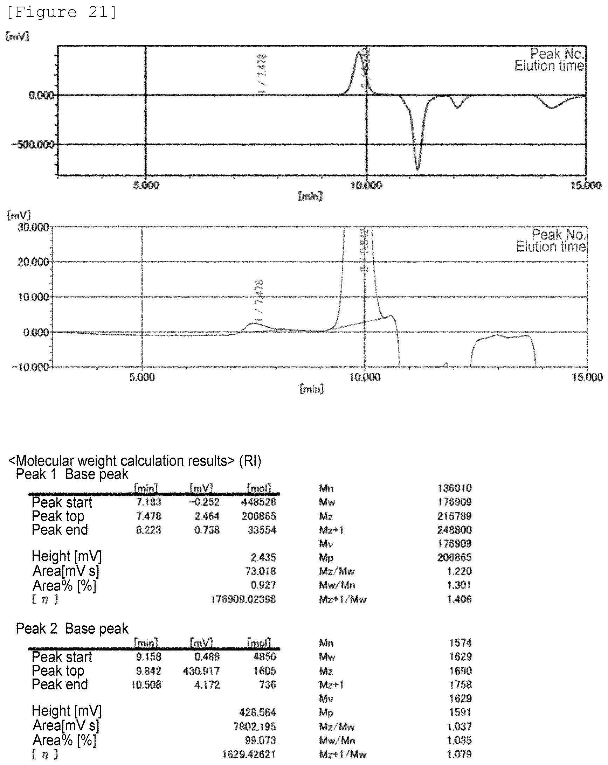

A portion of the coating liquid obtained as mentioned above was used as a sample for GPC, and the molecular weight of the acceptor was measured in accordance with (10) described above. The results of GPC measurement are shown in Figure 21. The weight-average molecular weight of the second component (high-molecular-weight component) was 176000, and the weight-average molecular weight of the first component (low-molecular-weight component) was 1600. The signal intensity of the second component (ratio of the peak area of the second component to the total peak area of the first component and the second component) was 0.93%.

In addition, the subsequent Examples and Comparative Examples were evaluated in the same way as the methods mentioned above. The results of each evaluation are also shown in Table 1.

### <Example 2>

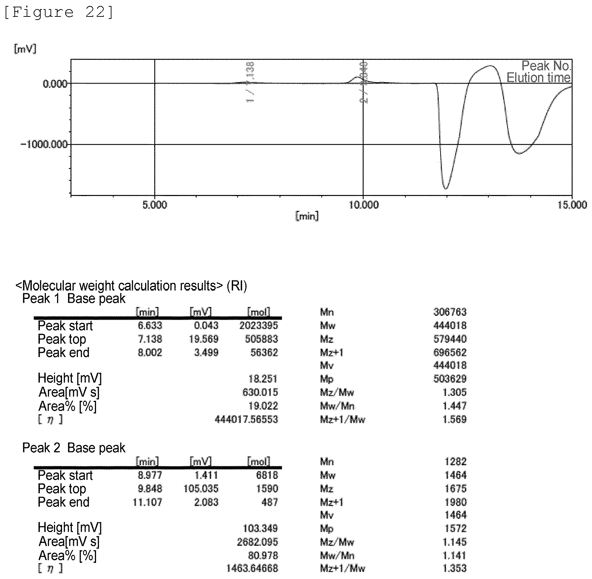

The procedures followed Example 1 except that 27.1 g of phenyltrimethoxysilane was changed to 13.5 g. The results of GPC measurement are shown in Figure 22. The weight-average molecular weight of the second component (high-molecular-weight component) was 444000, and the weight-average molecular weight of the first component (low-molecular-weight component) was 1500. The signal intensity of the second component (ratio of the peak area of the second component to the total peak area of the first component and the second component) was 19%.

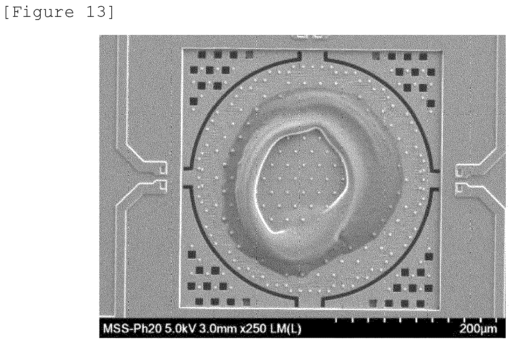

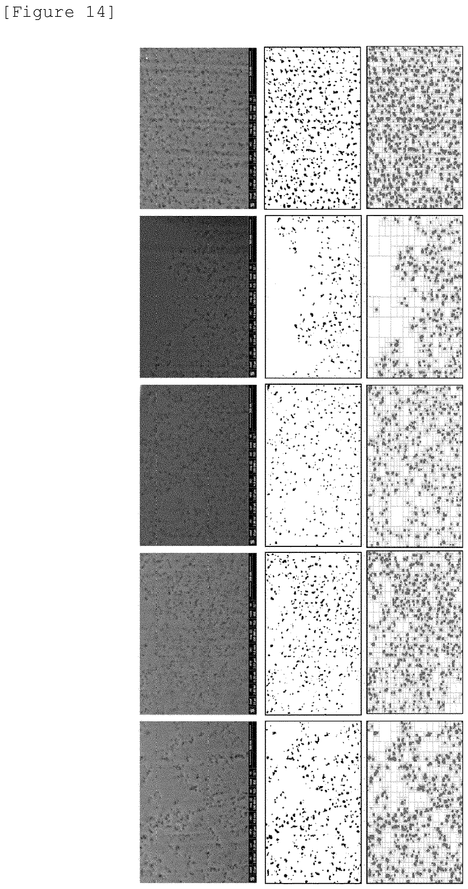

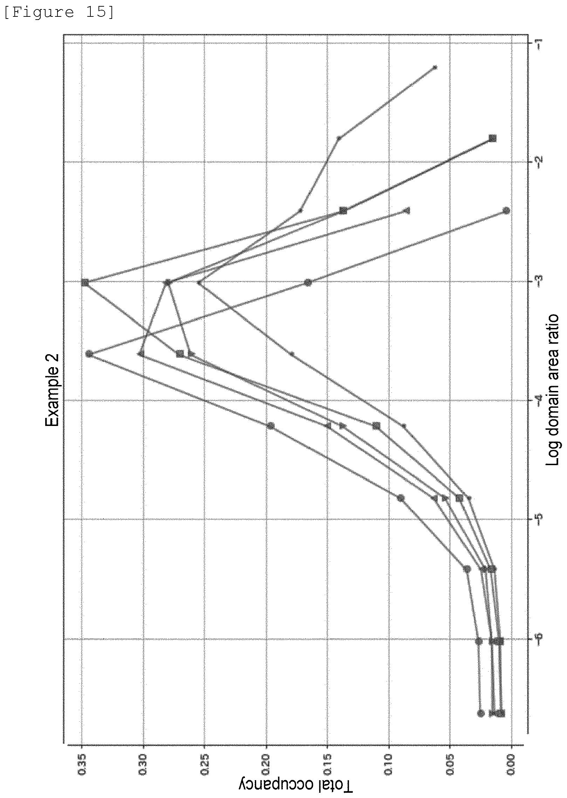

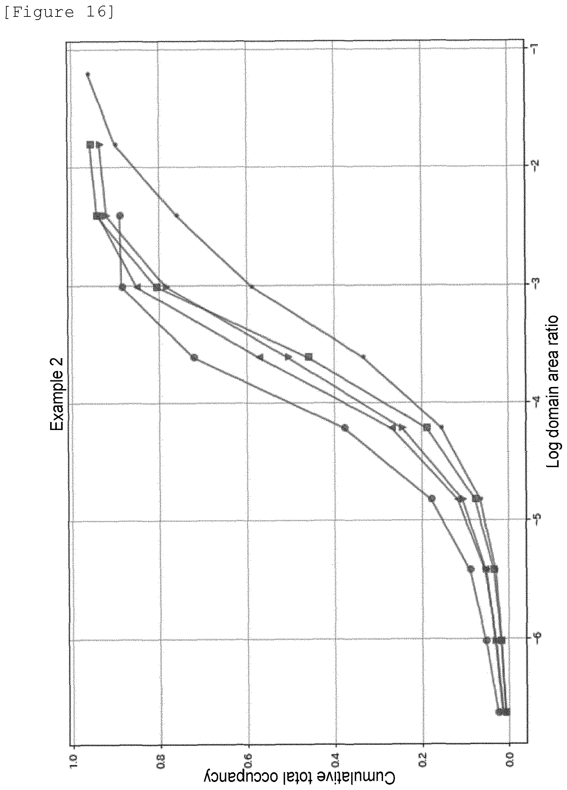

The surface of the acceptor in Example 2 was observed under SEM. The results are shown in Figure 13. SEM images of cross sections at arbitrary five points of the acceptor were obtained and binarized, followed by quadtree spatial partitioning by the method described above in (2) (Figure 14). The cross sections at arbitrary five points were adopted because domains where few voids were present and domains where voids were present were seen in Example 2. After the quadtree spatial partitioning, total occupancies and cumulative total occupancies were plotted against log area occupancies. As a result, the largest log area occupancy A and log area occupancy B of the five cross sections were -3.00 and -3.00, respectively (Figures 15 and 16). Thus, more voids than those in Example 1 were seen in the acceptor according to Example 2, with large variations among the five points, indicating that the voids had a distribution.

### <Example 3>

The procedures followed Example 1 except that 50 g of IPA-ST-UP (product of Nissan Chemical Corp.) was changed to 25 g of IPA-ST (12 nm spherical silica).

### <Example 4>

The procedures followed Example 1 except that 50 g of IPA-ST-UP (product of Nissan Chemical Corp.) was changed to 25 g of IPA-ST-L (45 nm spherical silica).

### <Example 5>

The procedures followed Example 1 except that 50 g of IPA-ST-UP (product of Nissan Chemical Corp.) was changed to 20 g of MP-4540M (450 nm spherical silica).

### <Example 6>

The procedures followed Example 1 except that 50 g of IPA-ST-UP (product of Nissan Chemical Corp.) was changed to 20 g of ZR-40BL (90 nm zirconia).

### <Example 7>

The procedures followed Example 1 except that 27.1 g of phenyltrimethoxysilane was changed to 37.6 g of 4-chlorophenyltriethoxysilane.

### <Example 8>

The procedures followed Example 1 except that 27.1 g of phenyltrimethoxysilane was changed to 30.4 g of 4-methoxyphenyltrimethoxysilane.

### <Example 9>

The procedures followed Example 1 except that 27.1 g of phenyltrimethoxysilane was changed to 31.5 g of 3-furyltriethoxysilane.

### <Example 10>

The procedures followed Example 1 except that 27.1 g of phenyltrimethoxysilane was changed to 33.9 g of 3-thienyltriethoxysilane.

### <Example 11>

The procedures followed Example 1 except that 27.1 g of phenyltrimethoxysilane was changed to 23.3 g of 4-aminophenyltrimethoxysilane.

### <Example 12>

The procedures followed Example 1 except that 27.1 g of phenyltrimethoxysilane was changed to 29 g of p-tolyltrimethoxysilane.

### <Example 13>

The procedures followed Example 1 except that 27.1 g of phenyltrimethoxysilane was changed to 27.2 g of naphthalenetrimethoxysilane.

### <Example 14>

The procedures followed Example 1 except that 27.1 g of phenyltrimethoxysilane was changed to 27.1 g of phenyltrimethoxysilane + 37.5 g of 3-(2-imidazolinepropyl)triethoxysilane.

### <Example 15>

The procedures followed Example 1 except that 27.1 g of phenyltrimethoxysilane was changed to 27.1 g of phenyltrimethoxysilane + 36.1 g of 3-(2-aminopropylethylamino)propyltriethoxysilane.

### <Example 16>

The procedures followed Example 1 except that 27.1 g of phenyltrimethoxysilane was changed to 27.1 g of phenyltrimethoxysilane + 35.7 g of norbornyltriethoxysilane.

### <Example 17>

The procedures followed Example 1 except that 27.1 g of phenyltrimethoxysilane was changed to 27.1 g of phenyltrimethoxysilane + 7.08 g of 3-N,N-dimethylaminopropyltrimethoxysilane.

### <Example 18>

The procedures followed Example 1 except that 27.1 g of phenyltrimethoxysilane was changed to 27.1 g of phenyltrimethoxysilane + 9.77 g of 3-mercaptopropyltriethoxysilane.

### <Example 19>

The procedures followed Example 1 except that 27.1 g of phenyltrimethoxysilane was changed to 24.4 g of phenyltrimethoxysilane + 3.93 g of pentafluorophenyltrimethoxysilane.

### <Example 20>

The procedures followed Example 1 except that 27.1 g of phenyltrimethoxysilane was changed to 27.1 g of phenyltrimethoxysilane + 11.9 g of 3-methacryloxypropyltriethoxysilane.

### <Example 21>

The procedures followed Example 1 except that 27.1 g of phenyltrimethoxysilane was changed to 27.1 g of phenyltrimethoxysilane + 8.75 g of 2-(3,4-epoxycyclohexyltrimethoxysilane.

### <Example 22>

The procedures followed Example 1 except that 27.1 g of phenyltrimethoxysilane was changed to 27.1 g of phenyltrimethoxysilane + 8.74 g of 4-aminophenyltrimethoxysilane.

### <Example 23>

The procedures followed Example 1 except that 27.1 g of phenyltrimethoxysilane was changed to 27.1 g of phenyltrimethoxysilane + 21.6 g of 3-ureidopropyltriethoxysilane.

### <Example 24>

The procedures followed Example 1 except that 27.1 g of phenyltrimethoxysilane was changed to 27.1 g of phenyltrimethoxysilane + 10.4 g of 3-phenylaminopropyltrimethoxysilane.

<Example 25>

The procedures followed Example 1 except that 27.1 g of phenyltrimethoxysilane was changed to 27.1 g of phenyltrimethoxysilane + 25.3 g of 3-[bis[(diphenylphosphino)methyl]amino]propyltrimethoxysila ne.

### <Example 26>

The procedures followed Example 8 except that 10.1 g of 3-furyltriethoxysilane was used.

### <Example 27>

The procedures followed Example 8 except that 17.3 g of 3-furyltriethoxysilane was used.

### <Example 28>

The surface of a sensor body for a MSS sensor disposed on a hot plate of 80°C was coated with 150 droplets of the coating liquid of Example 1 in an amount of 700 pL per droplet using an inkjet apparatus to obtain a MSS sensor equipped with an acceptor.

### <Example 29>

In a 500 mL separable flask, 6 g of phenyltrimethoxysilane was added to 18 g of water, 2 g of 28% ammonia water, and 80 g of 2-propanol, and the mixture was reacted at 80°C for 4 hours. The reaction solution was cooled in air and then centrifuged at 6000 rpm for 10 minutes to isolate precipitates. The sample was thoroughly washed with ethanol, dried, and then dissolved at 10 g/mL in DMF. A solution of dispersion IPA-ST-UP of chain-like colloidal silica in 2-propanol (product of Nissan Chemical Corp.) adjusted to 10 mg/mL with DMF, and the solution mentioned above were mixed at a ratio of 5:5 to obtain a coating liquid.

A portion of the coating liquid was used as a sample and dried at 100°C in the atmosphere as described above in (4), and its thermal weight reduction rate was measured.

The surface of a sensor body for a MSS sensor disposed on a hot plate of 40°C was coated with a portion of the coating liquid using an inkjet apparatus to obtain a MSS sensor equipped with an acceptor. In this coating, first, the surface was continuously coated with 300 pL each of two liquid droplets (600 pL) and dried for 1 second, and the same operation was repeated (a total of 30 times). Specifically, 18000 pL of the coating liquid was used.

### <Example 30>

The procedures followed Example 29 except that the mixing ratio of the solutions in Example 29 was changed from 5:5 to 1:9.

<Example 31>

In a 500 mL separable flask, 7.51 g of 4-chlorophenyltriethoxysilane was added to 18 g of water, 2 g of 28% ammonia water, and 80 g of 2-propanol, and the mixture was reacted at 80°C for 4 hours. The reaction solution was cooled in air and then centrifuged at 6000 rpm for 10 minutes to isolate precipitates. The sample was thoroughly washed with ethanol, dried, and then dissolved at 10 g/mL in DMF. A solution of dispersion TOL-ST of chain-like colloidal silica in toluene (manufactured by Nissan Chemical Corp.) adjusted to 10 mg/mL with DMF, and the solution mentioned above were mixed at a ratio of 5:5 to obtain a coating liquid.

A portion of the coating liquid was used as a sample and dried at 100°C in the atmosphere as described above in (4), and its thermal weight reduction rate was measured.

The surface of a sensor body for a MSS sensor disposed on a hot plate of 40°C was coated with a portion of the coating liquid using an inkjet apparatus to obtain a MSS sensor equipped with an acceptor. In this coating, first, the surface was continuously coated with 300 pL each of two liquid droplets (600 pL) and dried for 1 second, and the same operation was repeated (a total of 30 times). Specifically, 18000 pL of the coating liquid was used.

<Comparative Example 1>

Silica-titania particles (granular) were synthesized in accordance with the method described in WO2016/121155. The particles were synthesized by the cohydrolysis and polycondensation reaction of aminopropyltrimethoxysilane and titanium tetraisopropoxide (TTIP) in an aqueous solution of ammonia-basic isopropanol (IPA) containing octadecylamine (ODA) dissolved therein. The synthesis reaction was carried out using a Teflon(R) microreactor having a Y-shaped flow channel of micrometer size. Four precursor solutions were established: solution 1: aminopropyltrimethoxysilane/IPA, solution 2: H₂O/IPA/ammonia, solution 3: TTIP/IPA, and solution 4: H₂O/IPA. The solution 1 to the solution 4 were prepared such that their volumes were equal. The precursor solutions were sent at a constant rate at the same time using syringe pumps. The solution 1 with the solution 2 and the solution 3 with the solution 4 were mixed in parallel microreactors, respectively, and the solutions discharged from both the reactors were further mixed in another microreactor to prepare one reaction solution. The reaction solution was discharged into separately prepared precursor solution 5: ODA/H₂O/IPA, and stirred at a constant rate until the completion of the discharge. Then, the mixture was left standing at room temperature to obtain a nanoparticle (NH₂-STNP) dispersion. Since the nanoparticles were confirmed to have the structure represented by RSiO_{3/2}, this dispersion was treated as an organic-inorganic hybrid. The reaction solution was cooled in air and then centrifuged at 6000 rpm for 10 minutes to isolate precipitates. The sample was thoroughly washed with ethanol and then dissolved at 1 g/mL in N,N-dimethylformamide to obtain a coating liquid. The surface of a sensor body for a MSS sensor disposed on a hot plate of 80°C was coated with 300 droplets of the coating liquid in an amount of 300 pL per droplet using a microjet to obtain a MSS sensor equipped with an acceptor.

The microstructure of the central portion of the acceptor on the surface of the MSS sensor according to Comparative Example 1 was observed in the same way as in Example 1. The results are shown in Figure 6. Many
voids were observed in Comparative Example 1 compared with Example 1.

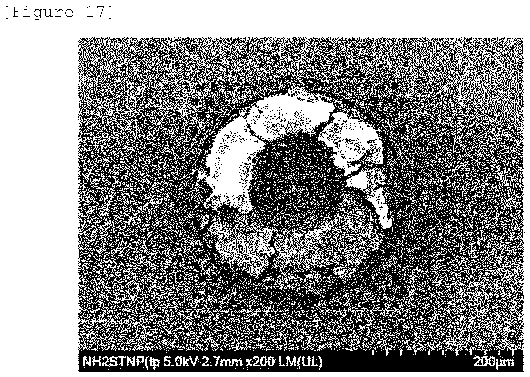

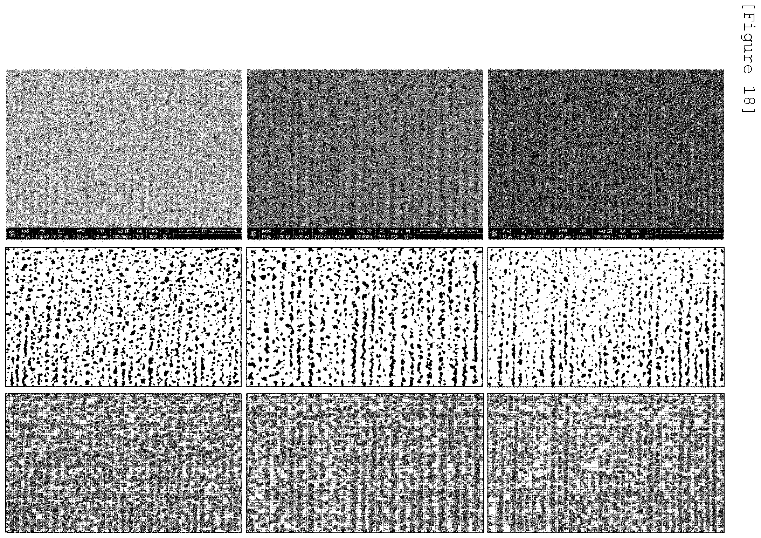

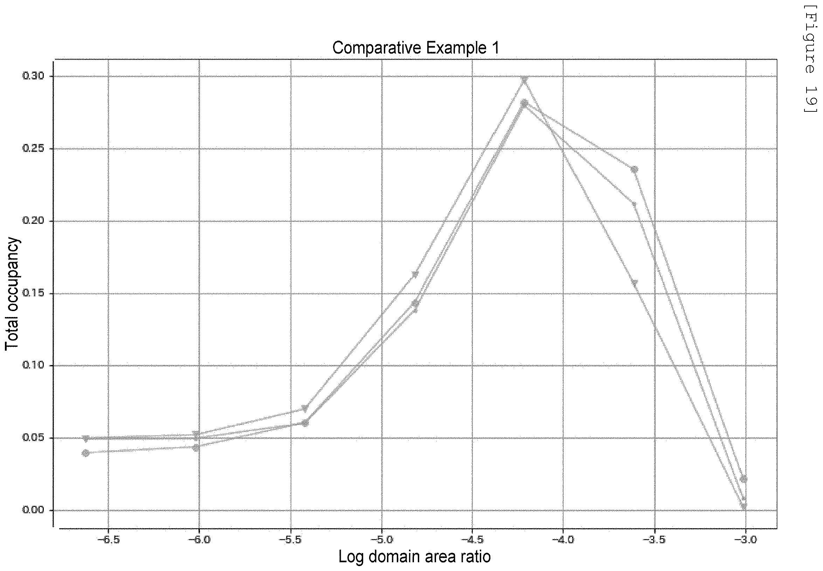

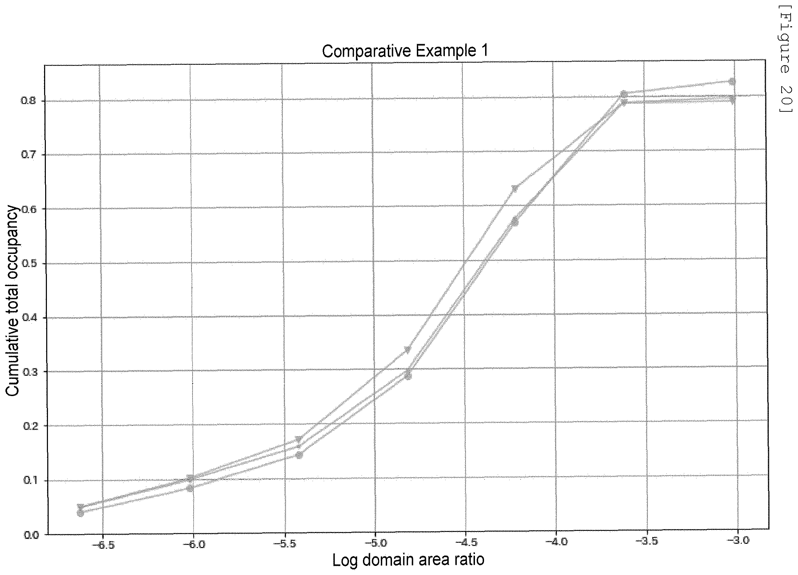

Aside from this, the surface of the acceptor according to Comparative Example 1 was observed under SEM. The results are shown in Figure 17. SEM images of cross sections at arbitrary three points of the acceptor were obtained and binarized, followed by quadtree spatial partitioning by the method described above in (2) (Figure 18). After the quadtree spatial partitioning, total occupancies and cumulative total occupancies were plotted against log area occupancies. As a result, the largest log area occupancy A and log area occupancy B of the three cross sections were -4.15 and -4.15, respectively (Figures 19 and 20). Thus, the acceptor according to Comparative Example 1 was found to be more porous with smaller voids uniformly dispersed, as compared with the acceptor according to Example 1.

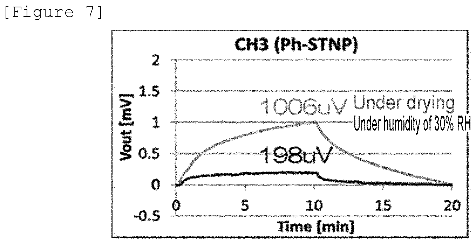

Signals of response to 100 ppm ethanol at a humidity of 0% or 30% were confirmed in the same way as in Example 1. The results are shown in Figure 7. Marked signal attenuation ascribable to the humidity of 30% was seen in Comparative Example 1.

### <Comparative Example 2>

The procedures followed Comparative Example 1 except that a nanoparticle (C₁₈-STNP) dispersion obtained using octadecyltriethoxysilane instead of aminopropyltrimethoxysilane was used. Since the nanoparticles were confirmed to have the structure represented by RSiO_{3/2}, this dispersion was treated as an organic-inorganic hybrid.

### <Comparative Example 3>

The procedures followed Comparative Example 1 except that a nanoparticle (phenyl-STNP) dispersion obtained using phenyltrimethoxysilane instead of aminopropyltrimethoxysilane was used. Since the nanoparticles were confirmed to have the structure represented by RSiO_{3/2}, this dispersion was treated as an organic-inorganic hybrid.

The results of performing the measurements (1) to (10) as to each of Examples and Comparative Examples are shown in the following Tables 1 to 3.

**[Table 1]**

| | | Example 1 | Example 2 | Example 3 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|---|
| (i) Inorganic filler | Type | Silica | Silica | Silica | Silica | Silica | Zirconia | Silica | Silica | Silica | Silica | Silica | Silica | Silica |
| | Shape | Chain-like | Chain-like | Spherical | Spherical | Spherical | Spherical | Chain-like | Chain-like | Chain-like | Chain-like | Chain-like | Chain-like | Chain-like |
| | Particle size (diameter) | 15 | 15 | 12 | 45 | 450 | 90 | 15 | 15 | 15 | 15 | 15 | 15 | 15 |
| (ii) Organic-inorganic hybrid | Organic functional group species | Phenyl | Phenyl | Phenyl | Phenyl | Phenyl | Phenyl | 4-Chlorophenyl | 4-Methoxyphenyl | 3-Furyl | 3-Thienyl | 4-Aminophenyl | p-Tolyl | 1-Naphthyl |
| | Amount of organic functional group | 45% | 20% | 28% | 46% | 28% | 30% | 61% | 44% | 37% | 41% | 36% | 54% | 66% |
| | RSiO_{3/2} structure | Present | Present | Present | Present | Present | Present | Present | Present | Present | Present | Present | Present | Present |
| Void parameter of acceptor | Void ratio | 0% | 8% | 2% | 0% | 2% | 3% | 0% | 1% | 1% | 1% | 1% | 0% | 0% |
| | Log domain area ratio A | -0.60 | -3.00 | -2.70 | -0.70 | -2.40 | -3.30 | -0.52 | -1.05 | -1.15 | -1.30 | -1.52 | -1.00 | 0.00 |
| | Log domain area ratio B | -1.10 | -3.00 | -2.70 | -1.00 | -2.40 | -3.22 | -1.05 | -1.05 | -1.19 | -1.30 | -1.70 | -1.05 | 0.00 |
| GPC measurement results of acceptor | First component | 1600 | 1500 | 1700 | 1700 | 1700 | 1600 | 2300 | 2500 | 1700 | 3000 | 2500 | 2200 | 1500 |
| | Second component | 176000 | 444000 | 170000 | 1000000 | 2000000 | 200000 | 200000 | 1160000 | 1660000 | 1830000 | 1450000 | 172000 | 200000 |
| | Signal intensity of high Mw in GPC (%) | 0.93 | 19 | 0.49 | 1 | 0.53 | 0.6 | 0.1 | 12.4 | 14.7 | 8,19 | 16.1 | 0.53 | 0.1 |
| Largest thickness of acceptor (µm) | | 3.8 | 5 | 0.2 | 12 | 27.3 | 3.5 | 6.7 | 5.2 | 8.7 | 10.2 | 9 | 15 | 10 |
| Evaluation results | Resistance to influence of humidity | A (40%) | B (43%) | A (40%) | A (38%) | A (40%) | A (32%) | S (10%) | A (32%) | A (40%) | A (34%) | B (50%) | S (9%) | S (7%) |
| | Physical or chemical stability | A | A | A | A | A | A | A | A | A | A | A | A | A |
| | Heat resistance | A | A | A | A | A | A | A | A | A | A | A | A | A |

**[Table 2]**

| | | Example 14 | Example 15 | Example 16 | Example 17 | Example 18 | Example 19 | Example 20 | Example 21 | Example 22 | Example 23 | Example 24 | Example 25 |
|---|---|---|---|---|---|---|---|---|---|---|---|---|---|
| (i) Inorganic filler | Type | Silica | Silica | Silica | Silica | Silica | Silica | Silica | Silica | Silica | Silica | Silica | Silica |
| | Shape | Chain-like | Chain-like | Chain-like | Chain-like | Chain-like | Chain-like | Chain-like | Chain-like | Chain-like | Chain-like | Chain-like | Chain-like |
| | Particle size (diameter) | 15 | 15 | 15 | 15 | 15 | 15 | 15 | 15 | 15 | 15 | 15 | 15 |
| (ii) Organic-inorganic hybrid | Organic functional group species | Phenyl +3-(2-imidazolinepropyl) | Phenyl +3-(2-aminopropylethylamino)propyl | Phenyl + norbornyl | Phenyl + 3-N,N-dimethylaminopropyl | Phenyl + 3-mercaptopropyl | Phenyl + pentafluorophenyl | Phenyl + 3-methacryloxypropyl | Phenyl + 2-(3,4-epoxycyclohexyl | Phenyl + 4-aminophenyl | Phenyl + 3-ureidopropyl | Phenyl + 3-phenylaminopropy l | Phenyl + 3-[bis[(diphenylphosphino) methyl]amino]propyl] |
| | Amount of organic functional group | 41% | 18% | 53% | 32% | 53% | 53% | 54% | 38% | 33% | 25% | 55% | 70% |
| | RSiO_{3/2} structure | Present | Present | Present | Present | Present | Present | Present | Present | Present | Present | Present | Present |
| Void parameter of acceptor | Void ratio | 3% | 4% | 1% | 5% | 0% | 0% | 0% | 0% | 0% | 1% | 1% | 0% |
| | Log domain area ratio A | -1.00 | -2.70 | -1.00 | -1.40 | -1.00 | -1.05 | -1.05 | -1.00 | -1.52 | -2.22 | -1.05 | 0.00 |
| | Log domain area ratio B | -1.05 | -2.52 | -1.00 | -1.40 | -1.00 | -1.05 | -1.07 | -1.00 | -1.40 | -2.22 | -1.00 | 0.00 |
| GPC measurement results of acceptor | First component | 1600 | 1600 | 9800 | 1500 | 4900 | 1700 | 1800 | 1800 | 1700 | 1600 | 1900 | 1500 |
| | Second component | 1570000 | 1820000 | 325000 | 1380000 | 170000 | 1640000 | 180000 | 1490000 | 1290000 | 1780000 | 354000 | 480000 |
| | Signal intensity of high Mw in GPC (%) | 18.5 | 45.1 | 1.35 | 51.6 | 1.5 | 4.6 | 0.08 | 8.96 | 167 | 26.4 | 0.15 | 1.57 |
| Largest thickness of acceptor (µm) | | 2.1 | 8.6 | 5.9 | 3.1 | 1.5 | 5.6 | 7.4 | 4.7 | 7.9 | 15.8 | 12.3 | 17.6 |
| Evaluation results | Resistance to influence of humidity | B (44%) | B (42%) | A (31%) | B (42%) | A (39%) | A (28%) | A (31%) | B (44%) | B (47%) | B (47%) | B (41%) | B (42%) |
| | Physical or chemical stability | A | A | A | A | A | A | A | A | A | A | A | A |
| | Heat resistance | A | A | A | A | A | A | A | A | A | A | A | A |

**[Table 3]**

| | | Example 26 | Example 27 | Example 28 | Example 29 | Example 30 | Example 31 | Comparative Example 1 | Comparative Example 2 | Comparative Example 3 |
|---|---|---|---|---|---|---|---|---|---|---|
| (i) Inorganic filler | Type | Silica | Silica | Silica | Silica | Silica | Silica | Absent | Absent | Absent |
| | Shape | Chain-like | Chain-like | Chain-like | Chain-like | Chain-like | Spherical | Absent | Absent | Absent |
| | Particle size (diameter) | 15 | 15 | 15 | 15 | 15 | 12 | - | - | - |
| (ii) Organic-inorganic hybrid | Organic functional group species | 3-Furyl | 3-Furyl | Phenyl | Phenyl | Phenyl | Chlorophenyl | 3-Aminopropyl | Octadecyl | Phenyl |
| | Amount of organic functional group | 10% | 21% | 50% | 27% | 49% | 33% | 22% | 70% | 49% |
| | RSiO_{3/2} structure | Present | Present | Present | Present | Present | Present | Present | Present | Present |
| Void parameter of acceptor | Void ratio | 8% | 3% | 0% | 4% | 1% | 0% | 15% | 11% | 11% |
| | Log domain area ratio A | -3.52 | -1.15 | -0.60 | -0.80 | -0.30 | -0.4 | -4.15 | -4.11 | -3.6 |
| | Log domain area ratio B | -3.40 | -1.19 | -1.10 | -1.20 | -0.40 | -0.3 | -4.15 | -3.85 | -4.12 |
| GPC measurement results of acceptor | First component | 1700 | 1800 | 1600 | 1700 | 1700 | 2300 | 1800 | 1600 | 1600 |
| | Second component | 1500000 | 1480000 | 176000 | 294000 | 34000 | 150000 | 0 | 0 | 0 |
| | Signal intensity of high Mw in GPC (%) | 30.5 | 21.3 | 0.93 | 7.74 | 0.02 | 8.52 | 0 | 0 | 0 |
| Largest thickness of acceptor (µm) | | 0.8 | 1.3 | 29.8 | 10.1 | 13.4 | 20.9 | 13.2 | 10.7 | 8.4 |
| Evaluation results | Resistance to influence of humidity | B (45%) | B (41%) | A (40%) | B (42%) | A (28%) | A (8%) | C (-94%) | C(85%) | C(89%) |
| | Physical or chemical stability | A | A | A | A | B | A | A | A | A |
| | Heat resistance | A | A | A | A | A | A | A | C | A |

The present application claims the priority to the Japanese patent application filed on February 8, 2019 (Japanese Patent Application No. 2019-021223), the Japanese patent application filed on August 29, 2019 (Japanese Patent Application No. 2019-156913), and the Japanese patent application filed on December 9, 2019 (Japanese Patent Application No. 2019-221949), the contents of which are incorporated herein by reference.

### Industrial Applicability

The sensor of the present invention can be used as an olfactory sensor in smell management or the like in breath tests, hotels, automobiles, or factories. The sensor of the present invention exerts, for example, an effect of exhibiting response to a hydrophilic gas such as ethanol or acetaldehyde with high sensitivity even in the presence of humidity and as such, can be suitably used in the field of transportation management of food products.

### Reference Signs List

- 1:: Sensor
- 2:: Acceptor (sensitive membrane)
- 3:: Chip (sensor body)

## Claims

1. A sensor comprising an acceptor to which a substance to be detected adheres,
wherein log domain area ratio A is -4.1 or more, the log domain area ratio providing a total occupancy maximum in a distribution of total occupancies against log domain area ratios calculated by subjecting the acceptor to the following measurement:
[Measurement]
In an image obtained by observing under a scanning electron microscope a surface or a cross section at an arbitrary position of the acceptor, quadtree spatial partitioning is performed in order to discriminate between void portions and dense portions of the surface or the cross section; a quadrangle group corresponding to the dense portions obtained by the quadtree spatial partitioning is classified on an area basis; and the log domain area ratio and the total occupancy are calculated as follows:
(Log domain area ratio)
A value determined by dividing each area of the quadrangle group by the area of the image is regarded as a domain area ratio, and a common logarithm thereof is taken as the log domain area ratio, and
(Total occupancy)
A value, which is represented by a ratio of a total area value of quadrangles having the same area in the quadrangle group to the area of the image being defined as 1, is regarded as the total occupancy.

2. The sensor according to claim 1, wherein the log domain area ratio A is -3.5 or more.

3. A sensor comprising an acceptor to which a substance to be detected adheres,
wherein log domain area ratio B is -4.1 or more, the log domain area ratio B providing a cumulative total occupancy of 60% in an ascending order of log domain area ratios in a distribution of total occupancies against log domain area ratios calculated by subjecting the acceptor to the following measurement:
[Measurement]
In an image obtained by observing under a scanning electron microscope a surface or a cross section at an arbitrary position of the acceptor, quadtree spatial partitioning is performed in order to discriminate between void portions and dense portions of the surface or the cross section; a quadrangle group corresponding to the dense portions obtained by the quadtree spatial partitioning is classified on an area basis; and the log domain area ratio and the total occupancy are calculated as follows:
(Log domain area ratio)
A value determined by dividing each area of the quadrangle group by the area of the image is regarded as a domain area ratio, and a common logarithm thereof is taken as the log domain area ratio, and
(Total occupancy)
A value, which is represented by a ratio of a total area value of quadrangles having the same area in the quadrangle group to the area of the image being defined as 1, and the resulting value is regarded as the total occupancy.

4. The sensor according to claim 3, wherein the log domain area ratio B is -3.5 or more.

5. The sensor according to any one of claims 1 to 4, wherein the acceptor comprises an organic-inorganic hybrid.

6. The sensor according to claim 5, wherein the organic-inorganic hybrid is represented by RSiO_{3/2} wherein the R represents an organic functional group.

7. A sensor comprising an acceptor to which a substance to be detected adheres, wherein
the acceptor comprises an organic-inorganic hybrid having a structure represented by RSiO_{3/2} wherein the R represents an organic functional group, and
a void ratio as a volume fraction of the acceptor is 10% or less.

8. The sensor according to claim 7, wherein the void ratio is 5% or less.

9. The sensor according to claim 7 or 8, wherein the void ratio is 3% or less.

10. A sensor comprising an acceptor to which a substance to be detected adheres,
wherein the acceptor comprises an organic-inorganic hybrid having a structure represented by RSiO_{3/2} wherein the R represents an organic functional group,
the acceptor comprises a first component having a weight-average molecular weight of 800 to 30000, and a second component having a weight-average molecular weight of 30000 to 2000000, and
in a chromatogram obtained by measuring the acceptor by gel permeation chromatography, a peak area of the second component is 0.01% to 55% with respect to a total peak area of the first component and the second component.

11. The sensor according to claim 10, wherein the acceptor further comprises a filler, and the second component is derived from the filler.

12. The sensor according to any one of claims 1 to 9, wherein the acceptor further comprises a filler.

13. The sensor according to claim 11 or 12, wherein the filler is an inorganic filler.

14. The sensor according to any one of claims 11 to 13, wherein the filler is a metal oxide.

15. The sensor according to any one of claims 11 to 14, wherein the filler is silica.

16. The sensor according to any one of claims 11 to 15, wherein a shape of the filler is a spherical shape or a chain-like shape.

17. The sensor according to any one of claims 6 to 16, wherein the organic functional group has an aromatic ring.

18. The sensor according to claim 17, wherein the aromatic ring is bonded directly to the Si atom in the organic-inorganic hybrid.

19. The sensor according to claim 17 or 18, wherein the organic functional group has a copolymer structure of the aromatic ring and a non-aromatic ring.

20. The sensor according to any one of claims 6 to 19, wherein the organic functional group has at least one atom selected from the group consisting of a halogen atom, a nitrogen atom, an oxygen atom, a phosphorus atom, a sulfur atom and a boron atom.

21. The sensor according to any one of claims 1 to 20, wherein an amount of the organic functional group in the organic-inorganic hybrid is 75% by mass or less.

22. The sensor according to any one of claims 1 to 21, wherein a largest thickness of the acceptor is 30 µmor smaller.

23. The sensor according to any one of claims 1 to 22, wherein the sensor is a membrane-type surface stress sensor.
